(19) Europäisches Patentamt / European Patent Office / Office européen des brevets

(11) **EP 4 201 938 A1**

(12) **EUROPEAN PATENT APPLICATION**
published in accordance with Art. 153(4) EPC


(43) Date of publication:
**28.06.2023 Bulletin 2023/26**

(21) Application number: **22755561.2**

(22) Date of filing: **17.02.2022**

(51) International Patent Classification (IPC):
***C07D 495/04*** *(2006.01)* ***A61K 31/4365*** *(2006.01)*
***A61P 3/10*** *(2006.01)*

(52) Cooperative Patent Classification (CPC):
**C07D 495/04; A61K 31/4365; A61P 3/10**

(86) International application number:
**PCT/CN2022/076637**

(87) International publication number:
**WO 2022/174788 (25.08.2022 Gazette 2022/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.02.2021 CN 202110194113**



(71) Applicants:
- **Lanzhou University**
  **Lanzhou, Gansu 730000 (CN)**
- **Tencent Technology (Shenzhen) Company Limited**
  **Shenzhen, Guangdong 518057 (CN)**

(72) Inventors:
- **BAI, Qifeng**
  **Lanzhou, Gansu 730099 (CN)**
- **XU, Tingyang**
  **Shenzhen, Guangdong 518057 (CN)**
- **FENG, Liya**
  **Lanzhou, Gansu 730099 (CN)**
- **LIU, Shuo**
  **Shenzhen, Guangdong 518057 (CN)**
- **HUANG, Junzhou**
  **Shenzhen, Guangdong 518057 (CN)**
- **LIU, Huanxiang**
  **Lanzhou, Gansu 730099 (CN)**
- **YAO, Xiaojun**
  **Lanzhou, Gansu 730099 (CN)**

(74) Representative: **Gunzelmann, Rainer**
**Wuesthoff & Wuesthoff**
**Patentanwälte PartG mbB**
**Schweigerstraße 2**
**81541 München (DE)**


(54) **GLUCAGON RECEPTOR ANTAGONIST AND USE THEREOF**


(57) The present disclosure relates to the field of medical technologies, and provides a compound and use thereof as a glucagon receptor antagonist. The compound includes a compound A, or an isomer, metabolite, prodrug, pharmaceutically acceptable ester, or pharmaceutically acceptable salt of the compound A. The glucagon receptor antagonist in the present disclosure has a novel skeleton structure and a low half maximal inhibitory concentration on the glucagon receptor.


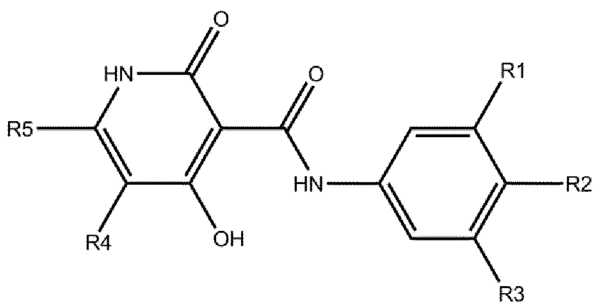
(A)

EP 4 201 938 A1

## Description

CROSS-REFERENCE TO RELATED APPLICATION

The present application claims priority to Chinese Patent Application No. 202110194113.X, filed on February 20, 2021, which is incorporated by reference in its entirety.

FIELD OF THE TECHNOLOGY

The present disclosure relates to the field of medical technologies, and specifically, to a glucagon receptor antagonist and use thereof.

BACKGROUND OF THE DISCLOSURE

Glucagon is a polypeptide hormone secreted by pancreatic islet $\alpha$ cells, consisting of 29 amino acids, mainly acting on the liver. By specifically binding to the glucagon receptor (GCGR) on the surface of liver cells, it can promote hepatic glycogenolysis and gluconeogenesis, causing an increase in blood sugar to counteract the hypoglycemic effect of insulin. The glucagon receptor is a G protein-coupled receptor that exists mainly on the surface of liver cells. It mainly binds to the glucagon to activate adenylate cyclase (AC) and phospholipase C (PLC), causing the concentration of cyclic adenosine monophosphate (cAMP) in the cells to increase, and activating cAMP-dependent protein kinase (PKA).

A glucagon receptor antagonist binds to the glucagon receptor to block the binding of glucagon to the glucagon receptor, inhibiting the function of glucagon, thereby reducing the response activity of cAMP, which is beneficial to lowering blood sugar level and regulating blood sugar balance.

At present, many glucagon receptor antagonists have been reported, but most of them have similar structures. There is a lack of structurally novel compounds as drug candidates. The information disclosed in the above background part is used only for enhancing the understanding of the background of the present disclosure.

SUMMARY

An objective of the present disclosure is to provide a structurally novel glucagon receptor antagonist and use of the glucagon receptor antagonist.

Other features and advantages of the present disclosure will be apparent through the following detailed description, or partly learned through practice of the present disclosure.

According to an aspect of the present disclosure, provided is a compound, represented by the following structural formula A, or an isomer, metabolite, prodrug, pharmaceutically acceptable ester, or pharmaceutically acceptable salt thereof,

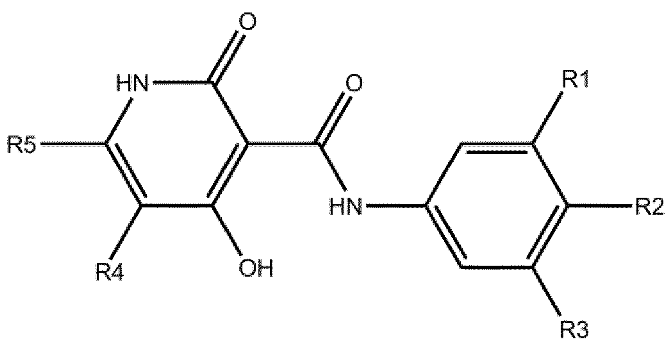

(A),

where R1 is H, F, Cl, Br, or I;

R2 is H or $-O-(CH_2)_m-CH_3$, m being an integer of 0-2;

R3 is H or $-S-(CH_2)_n-CH_3$, n being an integer of 0-2; and

R4 and R5 together form

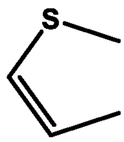 or 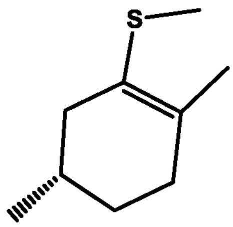 .

According to a specific embodiment of the present disclosure, the compound is a compound represented by the following structural formula A1 or A2:

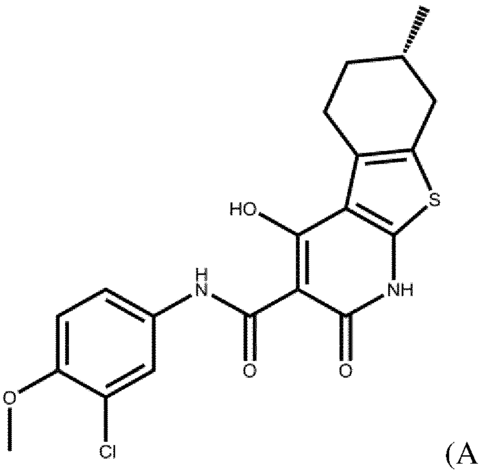
(A1) or
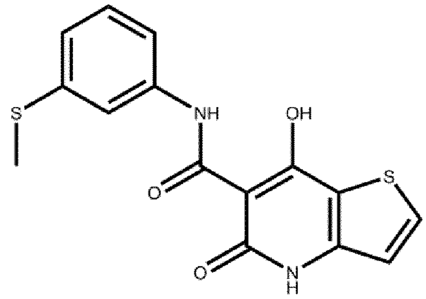
(A2).

According to an aspect of the present disclosure, a pharmaceutical composition is provided. The pharmaceutical composition includes the foregoing compound and a pharmaceutically acceptable carrier.

According to an aspect of the present disclosure, provided is use of the compound or the pharmaceutical composition in preparing a drug for use as a glucagon receptor antagonist.

The drugs are used for treating diseases associated with dysregulation of glucagon metabolism. Specifically, the compound is used for treating diabetes, and more specifically, for treating type I diabetes and/or type II diabetes; disorders of glucagon levels; and/or hyperglycemia.

The drugs are suitable for use in mammals, in particular, in human.

According to an aspect of the present disclosure, provided is a compound used as a glucagon receptor antagonist, defined above.

According to an implementation of the present disclosure, the compound is used for treating diseases associated with dysregulation of glucagon metabolism as defined above.

According to an aspect of the present disclosure, provided is a method for treating diseases associated with dysregulation of glucagon metabolism or regulating blood sugar levels, including administering an effective dose of the compound or the pharmaceutical composition to a subject.

The diseases associated with dysregulation of glucagon metabolism include diabetes (type I diabetes and/or type II diabetes); disorders of glucagon levels; and/or hyperglycemia.

The subject is a mammal, in particular, a human.

According to an aspect of the present disclosure, a glucagon receptor antagonist is provided. The glucagon receptor antagonist includes a compound A or an isomer, acid, ester, metabolite, prodrug, or pharmaceutically acceptable salt of the compound A; and the structural formula of the compound A is:

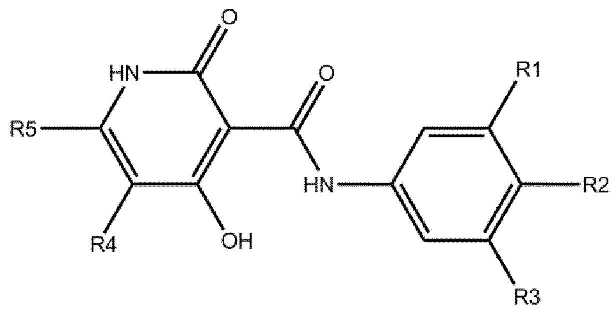

,

where R1 is independently ‖-H, ‖-F, ‖-Cl, ‖-Br, or ‖-I;
R2 is independently ‖-H or ‖-O-$(CH_2)_m$-$CH_3$, m being 0-2;
R3 is independently ‖-H or ‖-S-$(CH_2)$n-$CH_3$, n being 0-2;
R4 and R5 together form

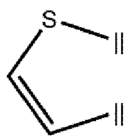 or 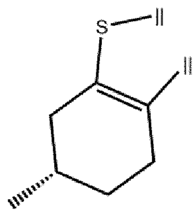 ;

and
‖ represents a point of attachment to a parent molecule.

According to an implementation of the present disclosure, R1 is ‖-Cl.

According to an implementation of the present disclosure, R2 is ‖-O-$CH_3$.

According to an implementation of the present disclosure, R3 is ‖-S-$CH_3$.

According to an embodiment of the present disclosure, R1 is ‖-Cl, R2 is ‖-O-$CH_3$, R3 is ‖-H, and R4 and R5 together form

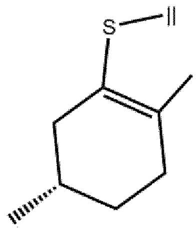

.

That is, the compound A is

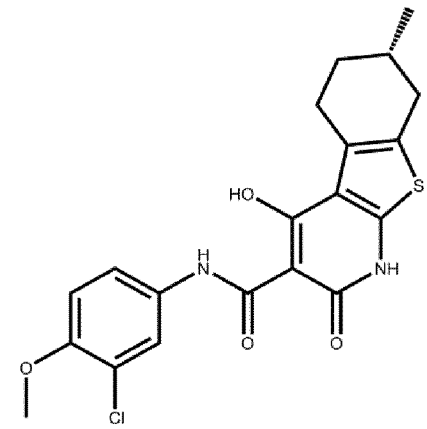

.

According to an embodiment of the present disclosure, R1 is ‖-H, R2 is ‖-H, R3 is ‖-S-$CH_3$, and R4 and R5 together form

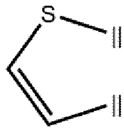

.

That is,
the compound A is

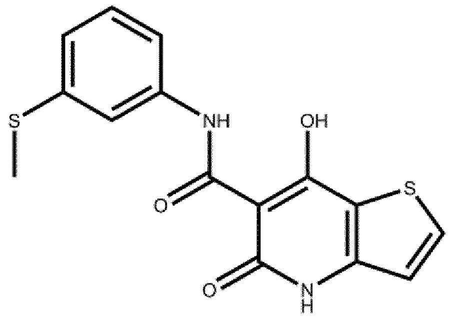

.

According to an aspect of the present disclosure, provided is use of a compound A in preparing drugs for treating diabetes or other diseases associated with dysregulation of glucagon metabolism. The structural formula of the compound A is:

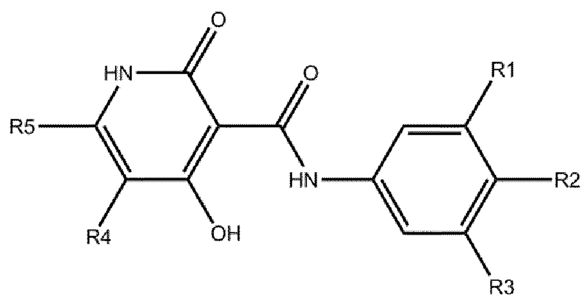

,

where R1 is independently ‖-H, ‖-F, ‖-Cl, ‖-Br, or ‖-I;

R2 is independently ‖-H or ‖-O-$(CH_2)_m$-$CH_3$, m being 0-2;

R3 is independently ‖-H or ‖-S-$(CH_2)$n-$CH_3$, n being 0-2;

R4 and R5 together form

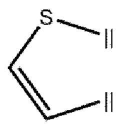 or 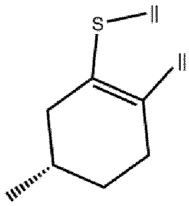 ;

and

‖ represents a point of attachment.

According to an aspect of the present disclosure, a pharmaceutical composition is provided, including a pharmaceutically acceptable carrier and the foregoing glucagon receptor antagonist.

According to some implementations of the present disclosure, the pharmaceutical composition is a solution, a tablet, a capsule, or an injection.

According to some implementations of the present disclosure, the pharmaceutical composition is administered by injection or orally.

According to an aspect of the present disclosure, a method for regulating a glucagon receptor in a subject is provided, including administering the foregoing glucagon receptor antagonist in a dose that inhibits the glucagon receptor to a subject in need thereof.

According to an aspect of the present disclosure, a method for regulating a glucagon receptor in a subject is provided, including administering the foregoing pharmaceutical composition in a dose that inhibits the glucagon receptor to a subject in need thereof.

In some embodiments of the present disclosure, the subject is a mammal.

According to an aspect of the present disclosure, a method for regulating blood sugar levels in a subject is provided, including administering the foregoing glucagon receptor antagonist in a dose that inhibits the glucagon receptor to a subject in need thereof.

According to an aspect of the present disclosure, a method for regulating blood sugar levels in a subject is provided, including administering the foregoing pharmaceutical composition in a dose that inhibits the glucagon receptor to a subject in need thereof.

In some embodiments of the present disclosure, the subject is a mammal.

According to an aspect of the present disclosure, provided is use of the foregoing glucagon receptor antagonist in preparing drugs for treating diabetes or other diseases associated with dysregulation of glucagon metabolism.

It can be learned from the foregoing technical solutions that the compound that can be used as a glucagon receptor antagonist in the exemplary embodiments of the present disclosure has at least the following advantages and positive effects.

The compound has a novel skeleton structure and a low half maximal inhibitory concentration on the glucagon receptor, and can be used as a promising candidate drug.

It is to be understood in the present disclosure that the above general descriptions and the following detailed descriptions are merely for exemplary and explanatory purposes, and cannot limit the present disclosure.

BRIEF DESCRIPTION OF THE DRAWINGS

The accompanying drawings herein, which are incorporated into the specification and constitute a part of this specification, show embodiments that conform to the present disclosure, and are used for describing a principle of this application together with the present disclosure. Apparently, the accompanying drawings in the following description show only some embodiments of the present disclosure, and a person of ordinary skill in the art may still derive other drawings from these accompanying drawings without creative efforts. In the accompanying drawings:

FIG. 1 shows the $^1$H-NMR spectrum of a compound A1.

FIG. 2 is a schematic 2D diagram of the interaction between the compound A1 and a glucagon receptor.

FIG. 3 shows the $^1$H-NMR spectrum of a compound A2.

FIG. 4 is a schematic 2D diagram of the interaction between the compound A2 in FIG. 3 and a glucagon receptor.

FIG. 5 is a curve graph showing the experimental results of the response of the compound A1 and the compound A2 to cAMP.

FIG. 6 is a schematic flowchart of a method for predicting an antagonist based on artificial intelligence according to an exemplary embodiment of the present disclosure.

FIG. 7 is a schematic flowchart of a method for predicting an antagonist based on artificial intelligence according to another exemplary embodiment of the present disclosure.

FIG. 8 is a schematic structural diagram of a generator network of an adversarial neural network according to an exemplary embodiment of the present disclosure.

FIG. 9 is a schematic structural diagram of a discriminator network of an adversarial neural network according to an exemplary embodiment of the present disclosure.

FIG. 10 is a schematic flowchart of a method for training a generator network of an adversarial neural network according to an exemplary embodiment of the present disclosure.

FIG. 11 is a schematic flowchart of a method for training a generator network of an adversarial neural network according to another exemplary embodiment of the present disclosure.

FIG. 12 is a schematic flowchart of a virtual screening method according to an exemplary embodiment of the present disclosure.

FIG. 13 is a schematic flowchart of an activity detection method of a target compound according to an exemplary embodiment of the present disclosure.

FIG. 14 is a curve graph showing the experimental results of the response of 30 compounds (where A corresponds to C1-C10, B corresponds to C11-C20, and C corresponds to C21-C30, with L-168,049 as a positive control) in Table 2 to cAMP.

DESCRIPTION OF EMBODIMENTS

Now, exemplary implementations are described comprehensively with reference to the accompanying drawings. However, the exemplary implementations may be implemented in various forms, and may not be understood as being limited to the examples described herein. Conversely, the implementations are provided to make the present disclosure more comprehensive and complete, and comprehensively convey the idea of the examples of the implementations to a person skilled in the art.

In addition, the described features, structures, or characteristics may be combined in one or more embodiments in any appropriate manner. In the following descriptions, a lot of specific details are provided to give a full understanding of the embodiments of the present disclosure. However, a person skilled in the art is to be aware of that, the technical solutions in the present disclosure may be implemented without one or more of the particular details, or other methods, unit, apparatus, or step may be adopted. In other cases, well-known methods, apparatuses, implementations, or operations are not shown or described in detail, to avoid obscuring the aspects of the present disclosure.

The flowcharts shown in the accompanying drawings are merely examples for descriptions, do not necessarily include all content and operations/steps, and are not necessarily performed in the described order. For example, some operations/steps may be further divided, while some operations/steps may be combined or partially combined. Therefore, an actual execution order may vary depending on an actual situation.

Glucagon and insulin are two hormones, with completely opposite effects, secreted by pancreatic islet cells. Glucagon is secreted by pancreatic islet $\alpha$ cells, and insulin is secreted by pancreatic islet $\beta$ cells. These two hormones interact and restrict each other to maintain blood glucose homeostasis. Glucagon acts to increase blood sugar. For example, excessive secretion of insulin leads to less secretion of glucagon, so that blood sugar decreases; conversely, excessive secretion of glucagon leads to less secretion of insulin, so that blood sugar increases.

Glucagon works with insulin to maintain normal blood sugar levels. Disturbing the balance of glucagon and

insulin levels can cause various diseases, such as diabetes and ketoacidosis.

After glucagon binds to the glucagon receptor, it converts the signal into the cell, and activates the phosphorylase of hepatocytes through the cAMP-PK system to accelerate glycogenolysis. Therefore, reducing the expression of the glucagon receptor can regulate blood sugar levels.

The glucagon receptor antagonist provided in the embodiments of the present disclosure specifically binds to the glucagon receptor to block the binding of the glucagon receptor to glucagon, so as to regulate blood sugar balance.

For this reason, the present disclosure provides a compound that can be used as the glucagon receptor antagonist.

The compound used as the glucagon receptor antagonist may be selected from the group consisting of a compound A with the following structural formula, an isomer, metabolite, prodrug, pharmaceutically acceptable ester, or pharmaceutically acceptable salt of the compound A. Specifically, the structural formula of the compound A is:

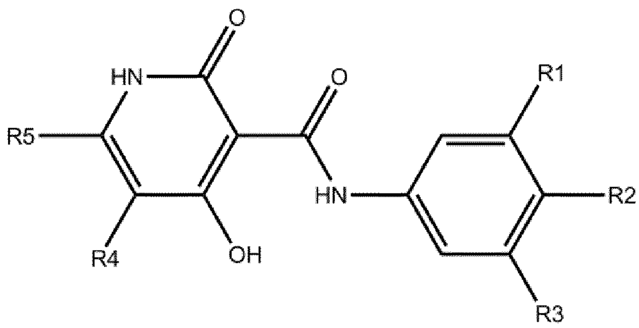
(A)

where,

R1 may be H, F, Cl, Br, or I. When R1 is H, there is no substitution at R1 on the benzene ring of the structural formula. When R1 is F, Cl, Br, or I, there is a substitution at R1 on the benzene ring of the structural formula. In some embodiments of the present disclosure, R1 is Cl or H.

R2 is H or $-O-(CH_2)_m-CH_3$, m being an integer of 0-2. When R2 is H, there is no substitution at R2 on the benzene ring of the structural formula.

When m is 0, R2 is $-O-CH_3$. When m is 1, R2 is $-O-CH_2-CH_3$. When m is 2, R2 is $-O-(CH_2)_2-CH_3$. In some embodiments of the present disclosure, R2 is H or $-O-CH_3$.

R3 is -H or $-S-(CH_2)_n-CH_3$, n being an integer of 0-2. When R3 is H, there is no substitution at R3 on the benzene ring of the structural formula.

When m is 0, R3 is $-S-CH_3$. When m is 1, R3 is $-S-CH_2-CH_3$. When m is 2, R3 is $-S-(CH_2)_2-CH_3$. In some embodiments of the present disclosure, R3 is H or $-S-CH_3$.

R4 and R5 together form

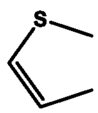 or 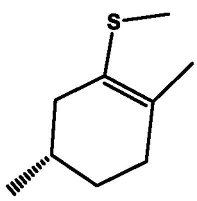 .

The isomer of the compound A includes an optical isomer and a geometric isomer.

The optical isomer is specifically, when R4 and R5 together form the chiral substituent

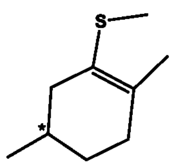 ,

not limited to

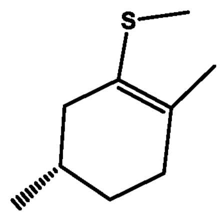

,

and may alternatively be

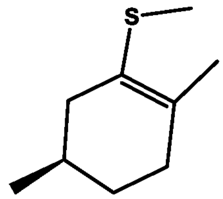

.

The geometric isomer refers to a cis-trans isomer in the present disclosure. Specifically, the geometric isomer in the present disclosure is formed by different arrangements of the double bonds of the parent molecule or the groups at the ring carbon atoms, and by different arrangements of the double bonds in the substituents of R4 and R5 or the groups at the ring carbon atoms.

The ester of the compound A may be obtained by reacting the compound A with organic acids. The ester has the functional group -COO-, and is obtained by dehydration condensation. For example, the organic acid reacts with -OH in the compound A to obtain the ester. The organic acid may be formic acid, acetic acid, propionic acid, or the like. The metabolite of the compound A refers to a product from which some functional groups are metabolized on the compound A. The metabolite refers to a substance produced or consumed by metabolism, and does not substantially affect the medicinal properties of the compound A.

The prodrug of the compound A, also referred to as pro-drug, a drug precursor, a precursor drug, and the like, refers to a compound which is obtained after chemical structure modification on the compound A, is inactive or less active in vitro, and releases the active drug through enzymatic or non-enzymatic conversion in vivo to achieve drug effects. That is, the prodrug of the compound A is generally a functional derivative of the compound A, which is readily converted to the compound A in vivo.

A solvate of the compound A refers to a form in which the compound A is diluted in a certain solvent. Common solvents include water, ethanol, dimethyl sulfoxide, and other liquid substances that can dissolve the compound A.

The pharmaceutically acceptable salt of the compound A includes salts formed in the following two ways.

(1) A salt is formed by the acidic functional group (such as -OH) in the compound A in the present disclosure with proper inorganic or organic cations (bases), such as a salt of the compound A in the present disclosure with an alkali metal or an alkaline earth metal, an ammonium salt of the compound A in the present disclosure, and a salt of the compound A in the present disclosure with a nitrogen-containing organic base. (2) A salt is formed by the basic functional group (such as -NH-) in the compound A in the present disclosure with proper inorganic or organic anions (acids), such as a salt of the compound in the present disclosure with an inorganic acid or an organic carboxylic acid.

Therefore, the pharmaceutically acceptable salt of the compound A in the present disclosure includes, but is not limited to, an alkali metal salt such as a sodium salt, a potassium salt, and a lithium salt; an alkaline earth metal salt such as a calcium salt and a magnesium salt; another metal salt such as an aluminum salt, an iron salt, a zinc salt, a copper salt, a nickel salt, and a cobalt salt; an inorganic alkali salt such as an ammonium salt; an organic alkali salt such as a tert-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-phenethylamine salt, a piperazine salt, a tetramethylamine salt, and a tris(hydroxymethyl)aminomethane salt; an inorganic acid salt, for example, a hydrohalic acid salt, such as a hydrofluoric acid salt, a hydrochloride salt, a hydrobromide salt, a hydroiodic acid salt, further, a nitrate, a perchlorate, a sulfate, and a phosphate; a lower alkane sulfonate salt such as a mesylate, a triflate, and an ethanesulfonate; an aryl sulfonate such as a benzenesulfonate and a p-benzenesulfonate; an organic carboxylate such as an acetate, a malate, a fumarate, a succinate, a citrate, a tartrate, an oxalate, and a maleate; and an amino acid salt such as a glycinate, a trimethylglycinate, an arginine salt, an ornithine salt, a glutamate, and an aspartate salt.

In an exemplary embodiment, R1 is -Cl, R2 is -O-$CH_3$, R3 is -H, and R4 and R5 together form

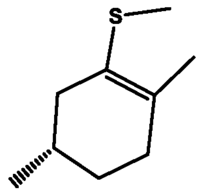

In this case, the compound A is:

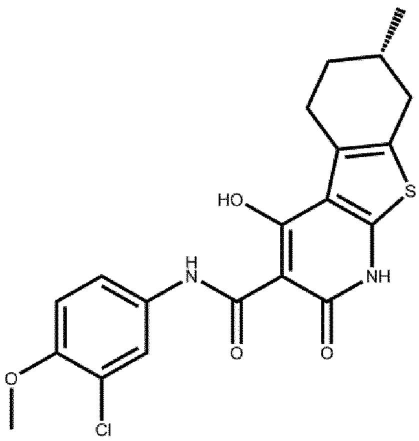

(A1),

denoted as compound A1.

The compound A1 has a molecular formula of $C_{20}H_{19}ClN_2O_4S$, with the name of (7S)-N-(3-chloro-4-methoxyphenyl)-4-hydroxy-7-methyl-2-oxo-5,6,7,8-tetrahydro-1H-[1]benzothi olo[2,3-b]pyridine-3-carboxamide.

InChI=1S/C20H19C1N204S/c1-9-3-5-11-14(7-9)28-20-15(11)17(24)16(19(26)23-2 0)18(25)22-10-4-6-13(27-2) 12(21)8-10/h4,6,8-9H, 3,5,7H2,1-2H3,(H,22,25)(H2,23,24,26)/t9-/m0/s 1.

The SMILES sequence is: COc1ccc(NC(=O)c2c(O)c3c4c(sc3[nH]c2=O)C[C@@H](C)CC4)cc1Cl.

The compound A1 may be prepared by any known method. Commercially available compound A1 is used in the following embodiments.

FIG. 1 shows the $^1$H NMR spectrum of the compound A1. The solvent is dimethyl sulfoxide (DMSO). This figure indicates that the molecular structure of the compound A1 is determined as the structure represented by the formula A1.

FIG. 2 is a schematic 2D diagram of the interaction between the compound A1 and a glucagon receptor. In this figure, ARG412, LYS405, ARG346, ASN404, SER350, THR353, LEU399, LEU403, LYS349, and GLN408 are amino acid residues in the amino acid sequence of the glucagon receptor. In the sequence of proteins, the amino and carboxyl groups between amino acids are dehydrated to form bonds. Some groups of the amino acid are involved in the formation of peptide bonds, so the remaining structural parts are referred to as amino acid residues.

LYS405, ARG346, SER350, and LYS349 all interact with the compound A1. When interacting, LYS405 and ARG346 are hydrogen donors and form hydrogen bonding interactions with the O atom on the compound A1. SER350 is a hydrogen acceptor and forms hydrogen bonding interactions with the H atom on the compound A1. LYS349 and $O^-$ in the compound A1 are attracted to each other through a salt bridge, that is, an ionic bond. Therefore, the above amino acid residues are all capable of interacting with the compound A1.

In addition, the mutation experiment reported in the relevant literature (Jazayeri A, Andrew S. Doré, Lamb D, et al. Extra-helical binding site of a glucagon receptor antagonist [J] Nature, 2016, May 12; 533(7602): 274-277) confirmed that LYS405, ARG346, SER350, and LYS349 are key residues in the binding pocket. The compound A1 in this embodiment can interact with the above residues, so the compound A1 well binds to the binding pocket.

In another exemplary embodiment, R1 is -H, R2 is -H, R3 is -S-$CH_3$, and R4 and R5 together form

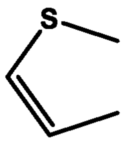

.

In this case, the compound A is:

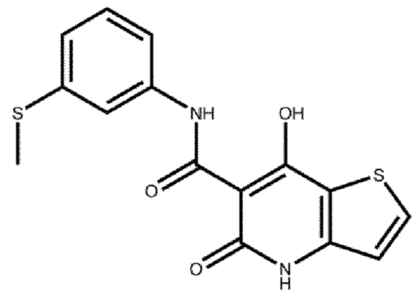

(A2),

denoted as compound A2.

The compound A2 has a molecular formula of $C_{15}H_{12}N_2O_3S_2$, with the name of 3-(azepan-1-ylcarbonyl)-N-(2,5-difluoro-phenyl)-7-methyl-1,8-naphthyridin-4-amine.

InChl=1S/C15H12N203S2/c1-21-9-4-2-3-8(7-9)16-14(19)11-12(18)13-10(5-6-22-1 3)17-15(11)20/h2-7H,1H3,(H,16,19)(H2,17,18,20).

The SMILES sequence is: CSc1cccc(NC(=O)c2c(O)c3sccc3[nH]c2=O)c1.

The compound A2 may be prepared by any known method. Commercially available compound A2 is used in the following embodiments.

FIG. 3 shows the $^1$H NMR spectrum of the compound A2. The solvent is dimethyl sulfoxide (DMSO). This figure indicates that the molecular structure of the compound A2 is determined as the formula A2.

FIG. 4 is a schematic 2D diagram of the interaction between the compound A2 and a glucagon receptor. In this figure, THR341, HIE340, ARG346, LYS405, ASN404, SER350, LEU399, GLN408, LYS349, and LEU403 are amino acid residues in the amino acid sequence of the glucagon receptor. ARG346, LYS405, SER350, and LYS349 all interact with the compound A2.

LYS405 and ARG346 are hydrogen donors and form hydrogen bonding interactions with the O atom on the compound A2. SER350 is a hydrogen acceptor and forms hydrogen bonding interactions with the H atom on the compound A2. LYS349 and $O^-$ in the compound A2 are attracted to each other through a salt bridge, that is, an ionic bond. Therefore, the above amino acid residues are all capable of interacting with the compound A2.

The compound A2 in this embodiment can interact with the above residues, so the compound A2 well binds to the binding pocket.

In order to determine the antagonistic activity of the compound A on the glucagon receptor in the above embodiments, a cell viability test is carried out. The specific implementation is as follows.

A known human glucagon receptor (hGCGR) antagonist drug is selected as a positive control, and the compound A in the above embodiments, a glucagon peptide segment (that is, human full-length glucagon), and human embryonic kidney cells containing target receptor molecules are mixed. The glucagon peptide segment binds to hGCGR to activate the hGCGR signaling pathway and release the second messenger cAMP. The compound A acts as an antagonist to inhibit the activity of hGCGR.

In an exemplary embodiment, a known hGCGR antagonist drug (L-168,049) is selected as a positive control. L-168,049 has the name of 4-[3-(5-bromo-2-propoxyphenyl)-5-(4-chlorophenyl)-1H-pyrrol-2-yl]pyridine, and is a noncompetitive glucagon receptor antagonist. In addition, in view of the human glucagon receptor on the cell membrane of human embryonic kidney cells HEK293, HEK293 is used to test the drug activity of the human glucagon antagonist. In this embodiment, different concentrations of compound A of the present disclosure and the positive control are respectively mixed with glucagon peptide segments and HEK293 cells, the second messenger cAMP is detected through homogeneous time resolved fluorescence (HTRF), and a half maximal inhibitory concentration value of each compound is calculated according to the intensity value of HTRF.

In an exemplary embodiment, the activity of the compound A is tested using the HTRF technique. Specifically, after activation of the glucagon receptor by the glucagon peptide segment, the second messenger cAMP is released from the glucagon receptor cells, the second messenger cAMP may be detected by HTRF, and the half maximal inhibitory concentration (IC50) of the tested compound may be calculated according to the fluorescence intensity.

In an exemplary embodiment, 11 identical concentration gradients are set for the compounds A1 and A2 respectively. The activities of the compounds A1 and A2 are determined by the HTRF second messenger cAMP response experiment in triplicate. The experimental data obtained is shown in Table 1.

Table 1

| | Compound A1 | | | Compound A2 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -5 | 98.36784 | 95.2799 | 96.14817 | 103.2945 | 102.1097 | 103.2736 | 100.4281 |
| -5.477121 | 87.84198 | 83.27731 | 83.50867 | 106.1285 | 105.9119 | 107.3553 | 98.6132 |
| -5.954243 | 75.44346 | 83.23603 | 79.30265 | 84.31701 | 92.47757 | 91.04928 | 98.05019 |
| -6.431364 | 54.65901 | 24.56393 | 41.98704 | 40.16475 | 62.73851 | 47.64906 | 93.04975 |
| -6.908485 | -13.6386 | 2.206529 | -0.67812 | 42.6475 | 14.48349 | 29.53025 | 89.45811 |
| -7.385606 | 35.8205 | 18.90853 | 45.6947 | 5.293268 | 31.48621 | 31.78721 | 47.8261 |
| -7.862728 | 7.61974 | -2.13549 | -0.78096 | -24.902 | 51.04536 | 18.00119 | 28.98286 |
| -8.339849 | 30.02611 | 27.22016 | 3.938939 | 33.24314 | -0.7378 | 10.67377 | 3.941144 |
| -8.81697 | -22.4972 | 19.34481 | 15.31759 | -19.9622 | 10.71795 | 11.92987 | -8.05055 |
| -9.294091 | 4.979932 | -28.5437 | 23.09991 | 15.16754 | 29.87882 | 11.08606 | -3.76273 |
| -9.771213 | 3.18964 | -35.8803 | -3.23056 | -4.69973 | 3.389463 | -14.8291 | -1.911 |

The data in Table 1 is processed through software using the concentration gradient as the abscissa and the inhibition percentage as the ordinate, to obtain FIG. 5. FIG. 5 is a curve graph showing the experimental results of the response of the tested compound to cAMP. Specifically, this figure shows the inhibition percentages of the responses of L-168,049, compound A1, and compound A2 respectively. L-168,049 is a positive control drug, the compound A1 is represented by C13, and the compound A2 is represented by C14. It can be seen from the figure that both compound A1 and compound A2 have good inhibitory activity of glucagon receptor.

Specifically, the test results are that IC50 of L-168,049 is 2.082E-08 M, IC50 of compound A1 is 4.465E-07 M, and IC50 of compound A2 is 4.225E-07 M.

The values of half maximal inhibitory concentration IC50 of compound A1 and compound A2 are small, which are 446.5 nM and 422.5 nM respectively, so they can be used as structurally novel glucagon receptor antagonist candidate drugs.

An embodiment of the present disclosure also provides use of the compound A in preparing drugs for treating diabetes or other diseases associated with dysregulation of glucagon metabolism.

The diseases associated with dysregulation of glucagon metabolism include, but are not limited to, diabetes (type I diabetes or type II diabetes), hyperglycemia, hyperinsulinemia, β-cell rest, abnormal β-cell function (that is, the compound can improve β-cell function by restoring the initial response), dietary hyperglycemia, apoptosis (that is, the compound can prevent apoptosis), impaired fasting glucose (IFG), metabolic syndrome, hypoglycemia, hyper/hypokalemia, disorder of glucagon levels (that is, the compound can normalize glucagon levels), dysregulation of the LDL/HDL ratio (that is, the compound can improve the LDL/HDL ratio), less snacks, eating disorder, weight loss, polycystic ovary syndrome (PCOS), obesity caused by diabetes, latent autoimmune diabetes (potentially admitted autoimmune diabetes), insulitis, islet transplantation, childhood diabetes, gestational diabetes, late complications of diabetes, micro/macro proteinuria, kidney disease, retinopathy, neuropathy, diabetic foot ulcers, decreased bowel motility due to glucagon administration, broken bowel syndrome, anti-diarrhea, increased gastric secretion, decreased blood flow, erectile dysfunction, glaucoma, post-operative stress, organ tissue damage caused by blood reperfusion after ischemia (that is, the compound can ameliorate organ tissue damage caused by blood reperfusion after ischemia), ischemic heart injury, insufficiency of heart function, congestive heart failure, stroke, myocardial infarction, arrhythmia, premature infant death, anti-apoptotic, wound healing, impaired glucose tolerance (IGT), insulin resistance syndrome, syndrome X, hyperlipidemia, dyslipidemia, hypertriglyceridemia, hyperlipoproteinemia, hypercholesterolemia, arteriosclerosis, and the like.

An embodiment of the present disclosure further provides a pharmaceutical composition including the foregoing glucagon receptor antagonist and a pharmaceutically acceptable carrier.

In this specification, the pharmaceutically acceptable carrier refers to a substance that is compatible with an individual recipient and suitable for delivering an active agent to a target without affecting the activity of the agent.

The pharmaceutical composition is suitable for use in mammals, further, in human. Unless otherwise specified, the following administration subjects are the same as those described herein.

The pharmaceutically acceptable carrier includes any and all solvents, diluents and other liquid vehicles, dispersion or suspension aids, surfactants, pH adjusting agents, isotonicity agents, thickening or emulsifying agents, preservatives, solid binders, lubricants, and the like, as appropriate for the particular dosage form desired.

Various carriers and known preparation techniques for formulating pharmaceutically acceptable compositions are disclosed by Remington: The Science and Practice of Pharmacy, 20th Edition, A.Gennaro, Lippincott Williams & Wilkins in 2000. Pharmaceutically acceptable excipients used in commercial products to dissolve compounds for oral or parenteral administration are reviewed by Strickley, Pharmaceutical Research, 21(2)201-230(2004). Any conventional carrier medium is encompassed within the scope of the present disclosure, unless it is incompatible with the glucagon receptor antagonist of the present disclosure, such as producing any undue biological effect or otherwise interacting in a detrimental manner with any other component of the pharmaceutically acceptable composition.

The pharmaceutically acceptable carrier includes (but is not limited to) an ion exchanger, aluminum oxide, aluminum stearate, lecithin, serum protein (such as human serum albumin), buffer (such as phosphate, carbonate, magnesium hydroxide, and aluminum hydroxide), glycine, sorbic acid or potassium sorbate, a mixture of partial glycerides of saturated vegetable fatty acids, water, pyrogen-free water, a salt or electrolyte (such as protamine sulfate, disodium hydrogen phosphate, potassium hydrogen phosphate, sodium chloride, and zinc salt), colloidal silica, magnesium trisilicate, polyvinylpyrrolidone, polyacrylate, wax, polyethylene-polyoxypropylene-block polymer, lanolin, sugar (such as lactose, glucose, sucrose, and mannitol), starch (such as cornstarch and potato starch), cellulose and its derivatives (such as sodium carboxymethylcellulose, ethylcellulose and cellulose acetate), powdered gum tragacanth, malt, gelatin, talc, excipient (such as cocoa butter and suppository wax), oil (such as peanut oil, cottonseed oil, safflower oil, sesame oil, olive oil, corn oil and soybean oil), diols (such as propylene glycol and polyethylene glycol), esters (such as ethyl oleate and ethyl laurate), agar, alginic acid, isotonic saline, Ringer's solution, alcohol (such as ethanol, isopropanol, cetyl alcohol, and glycerol), cyclodextrin (such as hydroxypropyl β-cyclodextrin and sulfobutyl ether β-cyclodextrin), lubricant (such as sodium lauryl sulfate and magnesium stearate), and petroleum hydrocarbon (mineral oil and paraffin oil). Colorants, release agents, coating agents, sweetening, flavoring and perfuming agents, preservatives and antioxidants may also be present in the composition, at the discretion of the formulator.

The pharmaceutical composition in the embodiments of the present disclosure may be prepared by methods well known in the art, such as conventional granulation, mixing, dissolving, encapsulation, lyophilization, or emulsification. The pharmaceutical composition may be produced in various forms, including granules, precipitates or microparticles, powders (including freeze-dried, spin-dried or spray-dried powders, amorphous powders), tablets, capsules, syrups, suppositories, injections, emulsions, elixirs, suspensions or solutions. The pharmaceutical composition may be administered by injection or orally.

An embodiment of the present disclosure further provides a method for regulating a glucagon receptor in a subject, including administering the foregoing glucagon receptor antagonist in a dose that inhibits the glucagon receptor to a subject in need thereof.

An embodiment of the present disclosure further provides a method for regulating a glucagon receptor in a subject, including administering the foregoing pharmaceutical composition in a dose that inhibits the glucagon receptor to a subject in need thereof.

The subject is a mammal, further, a human.

An embodiment of the present disclosure further provides a method for regulating blood sugar levels in a subject, including administering the foregoing glucagon receptor antagonist in a dose that inhibits the glucagon receptor to a subject in need thereof.

An embodiment of the present disclosure further provides a method for regulating blood sugar levels in a subject, including administering the foregoing pharmaceutical composition in a dose that inhibits the glucagon receptor to a subject in need thereof.

An embodiment of the present disclosure further provides use of the foregoing glucagon receptor antagonist in preparing drugs for treating diabetes or other diseases associated with dysregulation of glucagon metabolism.

The diseases associated with dysregulation of glucagon metabolism include, but are not limited to, diabetes (type I diabetes or type II diabetes), hyperglycemia, hyperinsulinemia, β-cell rest, abnormal β-cell function (that is, the compound can improve β-cell function by restoring the initial response), dietary hyperglycemia, apoptosis (that is, the compound can prevent apoptosis), impaired fasting glucose (IFG), metabolic syndrome, hypoglycemia, hyper/hypokalemia, disorder of glucagon levels (that is, the compound can normalize glucagon levels), dysregulation of the LDL/HDL ratio (that is, the compound can improve the LDL/HDL ratio), less snacks, eating disorder, weight loss, polycystic ovary syndrome (PCOS), obesity caused by diabetes, latent autoimmune diabetes (potentially admitted autoimmune diabetes, LADA), insulitis, islet transplantation, childhood diabetes, gestational diabetes, late complications of diabetes, micro/macro proteinuria, kidney disease, retinopathy, neuropathy, diabetic foot ulcers, decreased bowel motility due to glucagon administration, broken bowel syndrome, anti-diarrhea, increased gastric secretion, decreased blood flow, erectile dysfunction, glaucoma, post-operative stress, organ tissue damage caused by blood reperfusion after ischemia (that is, the compound can ameliorate organ tissue damage caused by blood reperfusion after ischemia), ischemic heart injury, insufficiency of heart function, congestive heart failure, stroke, myocardial infarction, arrhythmia, premature infant death, anti-apoptotic, wound healing, impaired glucose tolerance (IGT), insulin resistance syndrome, syndrome X, hyperlipidemia, dyslipidemia, hypertriglyceridemia, hyperlipoproteinemia, hypercholesterolemia, arteriosclerosis, and the like.

In another exemplary embodiment, screening may be carried out by a method for predicting an antagonist based on artificial intelligence to determine whether the glucagon receptor antagonist is the compound A1 or the compound A2.

The following specifically describes the method for predicting an antagonist based on artificial intelligence.

FIG. 6 is a schematic flowchart of a method for predicting an antagonist based on artificial intelligence according to an exemplary embodiment of the present disclosure. The method for predicting an antagonist based on artificial intelligence provided in this embodiment includes the following steps.

Step S210. Perform prediction processing on a molecular structure of a target drug-like molecule according to a generator network of a generative adversarial neural network obtained through training to obtain a simulated drug molecule. The generative adversarial neural network is trained according to a drug-like molecule and an existing antagonist corresponding to a target receptor molecule.

For example, the drug-like molecule and the existing antagonist corresponding to the target receptor molecule are used as a training sample of the generative adversarial neural network. The drug-like molecule in the training sample is inputted to the generator network in the generative adversarial neural network, and a molecular structure of the drug-like molecule is predicted based on the generator network, to obtain a predicted drug molecule. The predicted drug molecule, the drug-like molecule, and the existing antagonist are inputted to a discriminator network in the generative adversarial neural network, and parameters of the generative adversarial neural network are optimized according to an output of the discriminator network of the generative adversarial neural network, to obtain the trained generative adversarial neural network.

Based on the generative adversarial neural network obtained through training, a chemical formula of a target drug-like molecule is acquired for graph encoding to obtain corresponding graph representation, the graph representation

corresponding to the target drug-like molecule is inputted into the generator network of the generative adversarial neural network obtained through training, a molecular structure is predicted based on the generator network of the generative adversarial neural network obtained through training, and graph representation of the simulated drug molecule.

Step S220. Determine a small molecule database to be screened according to the simulated drug molecule.

For example, the two-dimensional simulated drug molecule is converted into a three-dimensional molecule by means of force field transformation of chemical simulation software, so as to obtain a small molecule database to be used as a drug database for virtual screening.

Step S230. Perform virtual screening on the small molecule database according to a target receptor molecule to obtain a target drug small molecule.

For example, using the target receptor molecule as a target, according to the shape characteristics, size characteristics, and electrostatic characteristics of a protein active pocket of the target, the affinity between the drug molecule in the small molecule database and the target is calculated by using an optimized search algorithm, and the target drug small molecule is screened out of the small molecule database as the target, that is, the target drug small molecule, corresponding to the target receptor molecule according to the affinity.

Step S240. Perform a cell viability test on the target drug small molecule to obtain a predicted antagonist of the target receptor molecule.

In the solution for predicting an antagonist based on artificial intelligence provided in the embodiment of FIG. 6, on the one hand, the simulated drug molecule of the target receptor molecule is acquired based on a deep learning model, to increase the diversity of the chemical space structure of the simulated drug molecule, so that the skeleton structure of the antagonist determined according to the simulated drug molecule is quite different from the skeleton structure of the existing drug, which is beneficial to improving the diversity of drugs. On the other hand, the small molecule database to be screened is determined according to the simulated drug molecule, and virtual screening is performed on the small molecule database according to the target receptor molecule, so that the target drug small molecule can directly search for a proper conformation in the protein active pocket of the target receptor, which is beneficial to ensuring the appropriateness of the designed antagonist.

The following exemplary embodiment is described by using human glucagon as the target receptor molecule. Certainly, the target receptor molecule may alternatively be a receptor of a characteristic type, which is not limited herein.

FIG. 7 is a schematic flowchart of a method for predicting an antagonist based on artificial intelligence according to another exemplary embodiment of the present disclosure. Referring to FIG. 7, the method for predicting an antagonist includes: a part 31 of simulated drug molecule acquisition, a part 32 of virtual screening of simulated drug molecule, and a part 33 of viability test on screened target drug small molecule. The part 31 of simulated drug molecule acquisition may be determined based on a generative adversarial network (GAN) obtained through training. For example, graph encoding is performed based on a chemical formula of a drug-like molecule to obtain graph representation corresponding to the drug-like molecule. For example, atoms are represented as points on the graph, and covalent bonds are represented as lines connecting the points. The graph representation corresponding to the drug-like molecule is inputted to a generator network of the GAN obtained through training, and the generator network of the GAN obtained through training may output graph representation corresponding to a simulated drug molecule.

In an exemplary embodiment, a network structure of GAN used in an exemplary embodiment of the present disclosure is first described. For example, FIG. 8 and FIG. 9 respectively show network structures of a generator network and a discriminator network of GAN according to an embodiment of the present disclosure.

Referring to FIG. 8, the generator network provided in this embodiment may include a convolution layer, a pooling layer, a feature supplementation layer, a deconvolution layer, and a feature normalization layer. In the neural network architecture, a convolution operation and a pooling operation may be used for extracting deep features of the graph representation of the drug-like molecule to learn a logical topological relationship in a molecular graph through the deep features. For example, the vertices of the molecular graph represent atoms, and the side lengths of the molecular graph represent the bond lengths of the molecule. The change rules of vertex and side length, and the relationship between vertex and side length are learned through model training. However, compared with the inputted graph representation of the drug-like molecule, multiple convolution operations and pooling operations reduce the feature image of the molecular graph, resulting in information loss. Therefore, in order to reduce the loss of information, for each down-sampling (such as 41-410 in FIG. 8), a corresponding up-sampling (that is, the feature supplementation layer and the deconvolution layer, such as 411-420 in FIG. 8) is performed. As a result, in the generator network, the up-sampling parameters correspond to the down-sampling parameters, so that the image is reduced in the up-sampling stage, and the corresponding image is enlarged in the down-sampling stage. That is, the generator network adopts the Unet network structure (the dotted line in the shape of "U" in FIG. 8) to reduce the loss of original information during network transmission, thereby reducing the phenomenon that the image content outputted by the generator network is inconsistent with the image content inputted to the generator network due to the mismatch between the down-sampling parameters and the up-sampling parameters, and finally increasing the prediction accuracy of the simulated drug molecule.

In an exemplary embodiment, in the generator network in FIG. 8, the tanh function may be used as an activation

function. For example, the output data of the up-sampling 420 may be mapped between -1 and 1 through the tanh function.

In an exemplary embodiment, referring to FIG. 9, a discriminator network of GAN provided in an exemplary embodiment of the present disclosure includes a plurality of convolution layers (such as 501-510 in FIG. 9). Wasserstein GAN (WGAN) may be used in this exemplary technical solution, so the discriminator network does not use the sigmoid function as the activation function in the down-sampling 510, thereby effectively avoiding the loss gradient of the generator network from disappearing.

In an exemplary embodiment, FIG. 8 and FIG. 9 only schematically show network structures of a generator network and a discriminator network. In actual operation, the network structure may be adjusted according to actual needs, so the network structure of the neural network for generating the simulated drug molecule in the present disclosure has scalability.

Based on the network structure of WGAN, FIG. 10 is a schematic flowchart of a method for training an adversarial neural network according to an exemplary embodiment of the present disclosure, including steps S610 to S630.

Step S610. Acquire a drug-like molecule and an existing antagonist corresponding to a target receptor molecule as a training sample of an adversarial neural network.

For example, when the target receptor molecule is a human glucagon receptor (hGCGR for short), an existing antagonist of hGCGR is acquired, such as an antagonist that has been reported; and a large number of drug-like molecules are acquired, for example, 1 million drug-like molecules are acquired in a commercial or open-source small molecule database (such as ZINC). The existing antagonist of hGCGR and the 1 million drug-like molecules are used as a training sample set.

Before the training sample is inputted to WGAN, graph encoding is first performed on the training sample to obtain corresponding graph representation. For example, in order to facilitate the processing in WGAN, the drug-like molecules and the existing antagonist in the training sample set are subjected to graph encoding one by one by means of adjacency tensor A and annotation matrix X. For example, the carbon atoms of a cyclopentane may be encoded as the vertices of a pentagon, and the sides of the pentagon correspond to the covalent bonds of the cyclopentane molecule (as shown in the part 31 of simulated drug molecule acquisition in FIG. 7).

Step S620. Input the drug-like molecule in the training sample to a generator network in the generative adversarial neural network, and predict a molecular structure of the drug-like molecule based on the generator network, to obtain a predicted drug molecule.

In an exemplary embodiment, FIG. 11 is a schematic framework diagram of a model training method according to an embodiment of the present disclosure. Referring to FIG. 11, the generative adversarial deep neural network includes a generator network G 710 and a discriminator network D 720.

The generator network G 710 is configured to perform prediction processing (such as convolution processing, deconvolution processing, and normalization processing) on a molecular structure of a drug-like molecule M (specifically, graph representation corresponding to the drug-like molecule M). An output of the generator network G 710 is a predicted drug molecule M' (specifically, graph representation corresponding to the predicted drug molecule M'). The generator network G 710 is trained to: perform prediction processing on the molecular structure of the drug-like molecule M to obtain the predicted drug molecule that makes the discrimination result of the discriminator network D 720 "true". That is, the discriminator network D 720 predicts that the probability that the predicted drug molecule M' is the antagonist of the target receptor molecule is greater than 0.5. That is, the generator network G 710 is trained to make the predicted drug molecule closer to the existing antagonist, so as to achieve an effect of "passing fake off as real" (the discriminator D 720 determines that the predicted drug molecule M' is the antagonist of the target receptor molecule as "true").

Further refer to FIG. 12. Step S630. Input the predicted drug molecule, the drug-like molecule, and the existing antagonist to a discriminator network in the generative adversarial deep neural network, and optimize parameters of the generative adversarial neural network according to an output of the discriminator network of the generative adversarial network.

In an exemplary embodiment, the predicted drug molecule M' (specifically the graph representation corresponding to the predicted drug molecule M') and the existing antagonist B (specifically the graph representation corresponding to the existing antagonist B) are inputted to the discriminator network D 720 to determine whether the predicted drug molecule M' is the antagonist of the target receptor molecule through the discriminator network D 720. For example, if the discriminator network D 720 determines that the predicted drug molecule M' is the antagonist of the target receptor molecule, indicating that the predicted drug molecule M' generated by the current generator has a high accuracy (that is, it can achieve an effect of "passing fake off as real"), then the optimization of the parameters of the generator network can be stopped. If the discriminator network D 720 determines that the predicted drug molecule M' is not the antagonist of the target receptor molecule, indicating that the predicted drug molecule M' generated by the current generator has an accuracy to be further increased (that is, it cannot achieve an effect of "passing fake off as real" so far), then the optimization of the parameters of the generator network needs to be continued.

In another exemplary embodiment, the output of the discriminator network includes a first discrimination result on the predicted drug molecule and a second discrimination result on the existing antagonist. The first discrimination

result includes a probability that the predicted drug molecule M' outputted by the discriminator network is the antagonist of the target receptor molecule. The second discrimination result includes a probability that the existing antagonist B outputted by the discriminator network is the antagonist of the target receptor molecule.

In an exemplary embodiment, further referring to FIG. 11, the discriminator network D 720 is configured to receive the predicted drug molecule M' (specifically the graph representation corresponding to the predicted drug molecule M') and the existing antagonist B (specifically the graph representation corresponding to the existing antagonist B). The discriminator network D 720 determines the probability that the predicted drug molecule M' is the antagonist of the target receptor molecule (that is, the first discrimination result) through a difference between the predicted drug molecule M' and the existing antagonist B. If the probability is greater than 0.5, the first discrimination result is that: the predicted drug molecule M' is the antagonist of the target receptor molecule, that is, the discrimination result outputted by the discriminator network is that: the predicted drug molecule M' is "true". Correspondingly, if the probability is not greater than 0.5, the first discrimination result is that: the predicted drug molecule M' is not the antagonist of the target receptor molecule, that is, the discrimination result outputted by the discriminator network is that: the predicted drug molecule M' is "false".

In addition, the discriminator network D 720 also predicts the probability that the existing antagonist B is the antagonist of the target receptor molecule (that is, the second discrimination result). If the probability is greater than 0.5, the second discrimination result is that: the existing antagonist B is the antagonist of the target receptor molecule, that is, the discrimination result outputted by the discriminator network is that: the existing antagonist B is "true". Correspondingly, if the probability is not greater than 0.5, the second discrimination result is that: the existing antagonist B is not the antagonist of the target receptor molecule, that is, the discrimination result outputted by the discriminator network is that: the existing antagonist B is "false".

The discriminator network D 720 is trained to: discriminate that the existing antagonist is "true" (the probability of the second discrimination result is greater than 0.5), and discriminate that the predicted drug molecule is "false" (the probability of the first discrimination result is not greater than 0.5).

In an exemplary embodiment, the parameters of the generative adversarial neural network are optimized according to the first discrimination result and the second discrimination result.

In an exemplary embodiment, further referring to FIG. 11, the first discrimination result and the second discrimination result are determined according to a logical topological difference between the predicted drug molecule M' and the existing antagonist B, and the parameters of the generator network G 710 and the parameters of the discriminator network D 720 are optimized according to the first discrimination result and the second discrimination result. For example, a loss function is represented by the formula (1):

$$\text{Loss} = \frac{1}{m}\sum_{i=1}^{m}\left[logD\left(x^i\right) + \log\left(1 - D\left(G(z^i)\right)\right)\right] \quad (1)$$

$x^i$ and $z^i$ respectively represent data corresponding to the existing antagonist B and data corresponding to the predicted drug molecule M', D represents the discriminator network D, G represents the generator network G, i is a positive integer that is not greater than m, and m is a sample number.

The loss function is optimized to $\min_G \max_D$ Loss. First, the parameters of the generator network G are fixed, and the parameters of the discriminator network D are adjusted. For example, the parameters of the discriminator D are optimized by the formula 2:

$$\max_D \text{Loss} = \frac{1}{m}\sum_{i=1}^{m}\left[logD\left(x^i\right) + \log\left(1 - D\left(G(z^i)\right)\right)\right] \quad (2)$$

$D(x^i)$ represents the second discrimination result (that is, the probability that the existing antagonist B is the antagonist of the target receptor molecule), the larger the value is, the better. $D(G(z^i))$ represents the first discrimination result (that is, the probability that the predicted drug molecule A' is the antagonist of the target receptor molecule), the smaller the value is, the better (that is, the larger $\log(1 - D(G(z^i)))$ is, the better).

For example, through $\max_D$ Loss, the first discrimination result outputted by the discriminator network D is that: the probability that the predicted drug molecule M' is the antagonist of the target receptor molecule is not greater than 0.5; and the second discrimination result is that: the probability that the existing antagonist B is the antagonist of the

target receptor molecule is greater than 0.5.

Then, the parameters of the discriminator D are fixed, and the parameters of the generator network G are adjusted. Since the generator network G does not involve the processing of the existing antagonist B (the corresponding data is $x^i$), $logD(x^i)$ is not involved in the process of optimizing the parameters of the generator network G. Specifically, the parameters of the generator G are optimized by the formula 3:

$$\min_G \text{Loss} = \frac{1}{m}\sum_{i=1}^{m}\log\left(1 - D\left(G(z^i)\right)\right) \quad (3)$$

$D(G(z^i))$ represents the first discrimination result (that is, the probability that the predicted drug molecule A' is the antagonist of the target receptor molecule), the larger the value is, the better (that is, the smaller $\log(1-D(G(z^i)))$ is, the better).

For example, after the predicted drug molecule A' outputted by the generator G with the parameters adjusted is inputted to the discriminator D through $\min_G$ Loss, the first discrimination result outputted by the discriminator D is that: the probability that the predicted drug molecule A' is the antagonist of the target receptor molecule is greater than 0.5.

After continuous iterative calculation, the above loss value satisfies a preset requirement. In addition, the generator network G (also referred to as molecular graph growth model) of GAN obtained through training may grow a simulated drug molecule that is similar to that in the training set.

In an exemplary embodiment, when the model prediction is performed by using WGAN, an earth mover (EM) distance may be used as a loss function. Since the log value is no longer calculated in the loss function, the training can be more easily converged.

The specific implementation of determining the simulated drug molecule based on the molecular graph growth model obtained through training (that is, the generator network of GAN or WGAN obtained through training) is as follows. A chemical formula of a target drug-like molecule is acquired for graph encoding to obtain corresponding graph representation; and the graph representation corresponding to the target drug-like molecule is inputted into the molecular graph growth model, and a molecular structure is predicted based on the molecular graph growth model, to output graph representation of the simulated drug molecule.

The molecular graph growth model outputs the graph representation corresponding to the simulated drug molecule. In order to facilitate the subsequent virtual screening, decoding processing needs to be performed to obtain a standard format of the simulated drug molecule, such as a linear symbol for inputting and representing a molecular reaction (simplified molecular input line entry system, SMILES) and a structure data file (SDF).

Therefore, the encoded simulated drug molecule outputted by the molecular graph growth model is decoded to obtain the chemical formula of the simulated drug molecule that satisfies a preset format.

Further referring to FIG. 7, after the part 31 of simulated drug molecule acquisition, there is the part 32 of virtual screening of simulated drug molecule. The following describes the embodiment of virtual screening of the simulated drug molecule.

For example, further referring to FIG. 6, in step S220, a small molecule database to be screened is determined according to the simulated drug molecule; and in step S230, virtual screening is performed on the small molecule database according to a target receptor molecule to obtain a target drug small molecule.

The small molecule database is a database for virtual screening. For example, 10 million simulated drug molecules are generated by using the molecular graph growth model and decoded to obtain standard formats of these simulated drug molecules. In order to search for a proper conformation in the protein active pocket by the simulated drug molecule directly, it is necessary to convert the simulated drug molecule into a three-dimensional simulated drug molecule (for example, a mol2 format). In an exemplary embodiment, the two-dimensional simulated drug molecule is converted into a three-dimensional molecule by means of force field transformation of chemical simulation software, so as to obtain the small molecule database to be used as a drug database for virtual screening.

In an exemplary embodiment, for comparison, 100,000 non-simulated drug molecules may be randomly selected from an existing database (for example, the ChemBridge database). The non-simulated drug molecules are merged into the small molecule database to be used as a drug database for virtual screening.

In an exemplary embodiment, in view of the principle of virtual screening, candidate compounds that can be used as targets are screened out of the small molecule database according to the shape characteristics, size characteristics, and electrostatic characteristics of the protein active pocket of the target (target receptor molecule). Therefore, before virtual screening is performed on the new molecular database, in order to ensure the smooth progress of the virtual screening, the target receptor molecule needs to be preprocessed, such as completion of amino acid residues in the target receptor molecule, hydrogenation of the target receptor molecule, and charging of the target receptor molecule.

For example, the human glucagon receptor molecule hGCGR (number: 5EE7) is downloaded from an existing database (such as the PDB database) as the target receptor molecule. The hGCGR is preprocessed as follows. The amino acid residues of the hGCGR protein molecule are completed by using the Schrödinger software, the target receptor molecule hGCGR is subjected to hydrogenation and charging, and the preprocessed hGCGR protein molecule is saved in a three-dimensional format (for example, mol2 format).

In order to ensure the smooth progress of the virtual screening, the format of the target receptor molecule and the format of the drug molecule in the small molecule database are unified, for example, they are unified into the mol2 format.

In an exemplary embodiment, referring to FIG. 12, based on the molecular docking technology, a virtual screening process is performed on the small molecule database and the target receptor molecule in the three-dimensional format. The molecular docking technology is based on the principle of the "lock and key" model. A receptor protein 81 is equivalent to a "lock", and a small molecule 82 is equivalent to a "key". The virtual screening is implemented by whether the two can be combined into 83. For example, using the target receptor molecule hGCGR as a target, according to the shape characteristics, size characteristics, and electrostatic characteristics of a protein active pocket of the target, the affinity between the drug molecule in the small molecule database and the target is calculated by using an optimized search algorithm, and the target drug small molecule is screened out of the small molecule database as the target, that is, the candidate compound, corresponding to the target receptor molecule hGCGR according to the affinity.

For example, depending on whether a small molecule in the small molecule database is a potential target drug, each small molecule is scored through a scoring function, the small molecules are sorted in descending order according to the scores, and the first multiple (for example, 30) small molecules in a descending sorting result are used as the target drug small molecules, see Table 2.

Table 2

| Number | SMILES sequence of target drug small molecule |
|---|---|
| C1 | NS(=O)(=O)c1ccc(NC(=S)NNC(=S)NC2CCCCC2)cc1 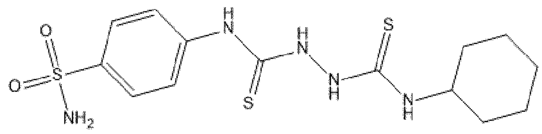 |
| C2 | O=S(=O)(NCCc1ccccc1)c1ccc(NC(=S)Nc2ccccc2)cc1 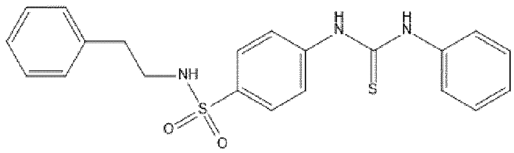 |
| C3 | COc1ccc(C(=O)NC(=S)Nc2ccc(NC(=S)NC(C)=O)cc2)cc1Cl 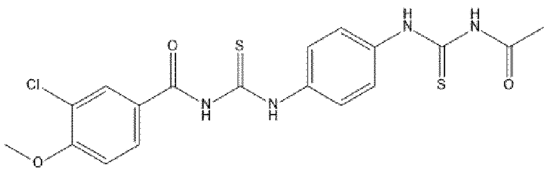 |
| C4 | O=S(=O)(Nc1ccc(Cl)cc1)c1ccc(NC(=S)Nc2ccccc2)cc1 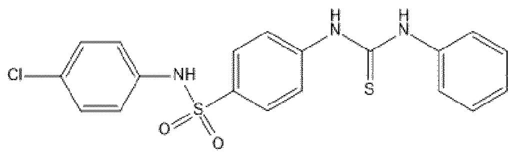 |

(continued)

| Number | SMILES sequence of target drug small molecule |
|---|---|
| C5 | O=C(NCCCCCCNC(=O)NCc1cccnc1)NCc1cccnc1<br>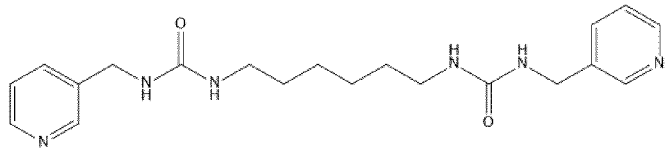 |
| C6 | O=C(CCCCC(=O)NNC(=S)Nc1ccc(Br)cc1)NNC(=S)Nc1ccc(Br)cc1<br>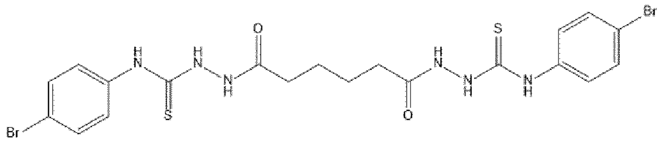 |
| C7 | O=C(NNC(=S)Nc1ccccc1)[C@H](O)[C@@H](O)C(=O)NNC(=S)Nc1ccccc1<br>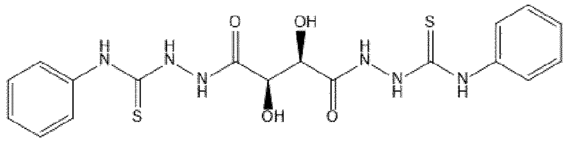 |
| C8 | CCC(=O)NC(=S)Nc1ccc(NC(=S)NC(=O)c2ccccc2OC)cc1<br>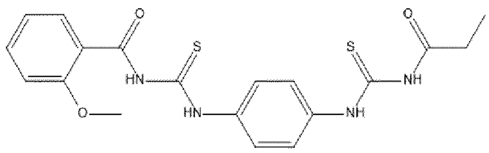 |
| C9 | CCC(=O)NC(=S)Nc1ccc(NC(=S)NC(=O)c2cccc(OC)c2)cc1<br>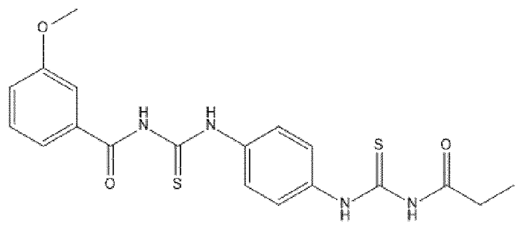 |
| C10 | CN1C(=O)Cc2cc(S(=O)(=O)CCC(=O)NCCc3ccccc3)ccc21<br>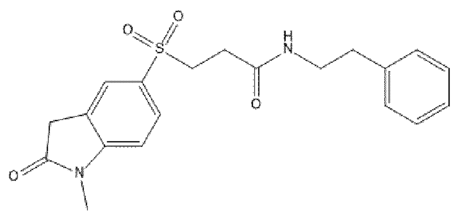 |
| C11 | CC(=O)c1ccccc1NC(=O)c1c(O)c2c3c(sc2[nH]c1=O)C[C@H](C)CC3<br>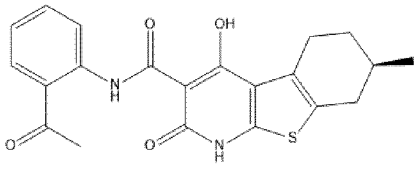 |

(continued)

| Number | SMILES sequence of target drug small molecule |
|---|---|
| C12 | COc1cc(/C=N/NC(=O)CN2C@H]3NC(=O)N[C@@H]32)cc(OC)c1O<br>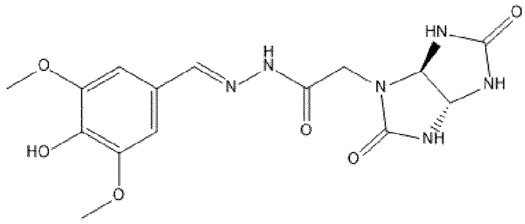 |
| C13 | COc1ccc(NC(=O)c2c(O)c3c4c(sc3[nH]c2=O)C[C@@H](C)CC4)cc1C1<br>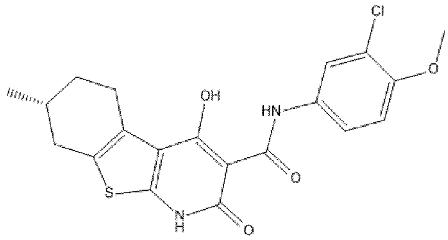 |
| C14 | CSc1cccc(NC(=O)c2c(O)c3sccc3[nH]c2=O)c1<br>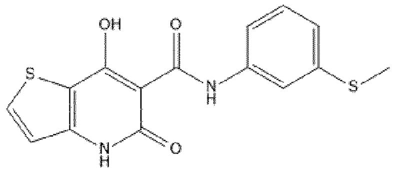 |
| C15 | COc1ccc2cc(C(=O)NCCNc3ccc(C)nn3)[nH]c2c1<br>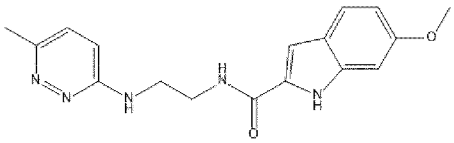 |
| C16 | O=C(CCNC(=O)c1ccccc1)NCc1cccc(NC(=O)C2CC2)c1<br>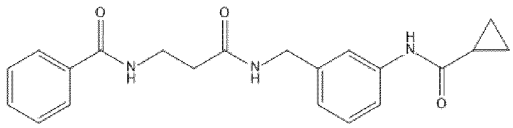 |
| C17 | CSc1cccc(NC(=O)CCNS(=O)(=O)c2ccc(NC(C)=O)cc2)c1<br>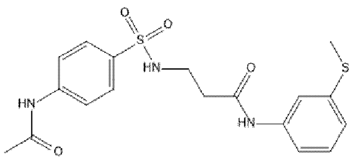 |
| C18 | O=C(COC(=O)CCNS(=O)(=O)c1ccc(C1)cc1)NC(=O)NCc1ccco1<br>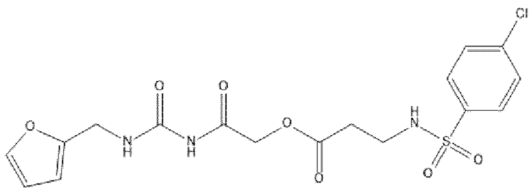 |

(continued)

| Number | SMILES sequence of target drug small molecule |
|---|---|
| C19 | O=C(CNC(=O)NCCCOC1CCCCC1)Nc1ccc(F)c(F)c1 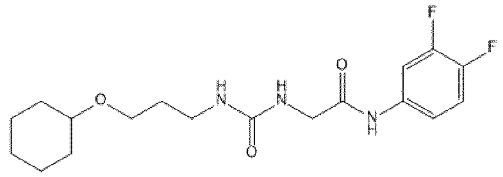 |
| C20 | O=C(COC(=O)CCNC(=O)c1ccsc1)NC(=O)NCc1cccs1 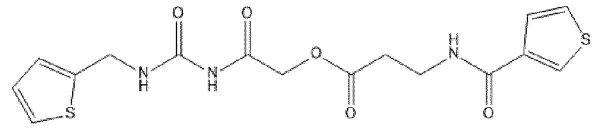 |
| C21 | O=C(CSc1nc2ccccc2[nH]1)NC(=O)Nc1ccc2c(c1)OCO2 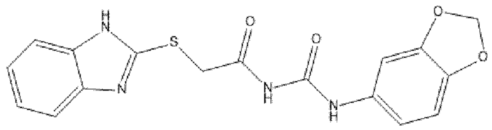 |
| C22 | O=C(CNC(=O)NCCCN1CCCCCC1=O)Nc1ccc(F)c(F)c1 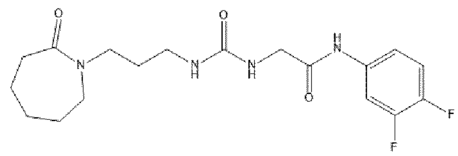 |
| C23 | Cc1ccc(-c2csc(NC(=O)c3ccc4c(c3)NC(=O)CS4)n2)c(C)c1 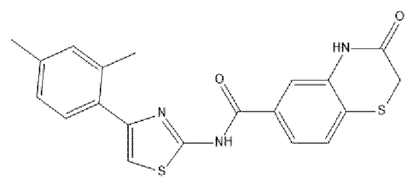 |
| C24 | CNC(=O)Cc1ccccc1NC(=O)NCCC(=O)NC1CCCC1 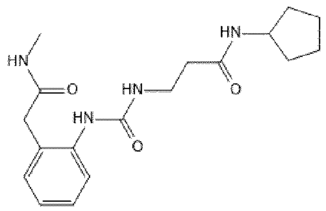 |
| C25 | CC(C)(C)NC(=O)NC(=O)CSc1nc(-c2cccs2)c(-c2cccs2)[nH]1 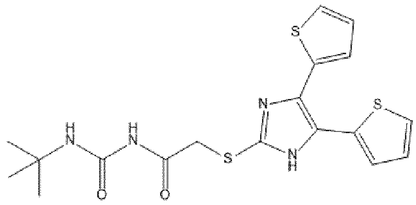 |

(continued)

| Number | SMILES sequence of target drug small molecule |
|---|---|
| C26 | COc1cc(NC(=O)CNC(=O)CNC(=O)c2cccs2)c(OC)cc1Cl 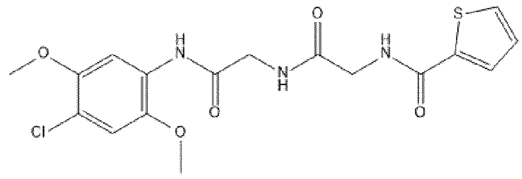 |
| C27 | O=C(CCNC(=O)NC1CCCCC1)NNc1ccccc1C(F)(F)F 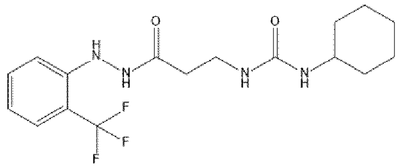 |
| C28 | CS(=O)(=O)NCCOc1ccc(S(=O)(=O)NCCOc2ccccc2)cc1 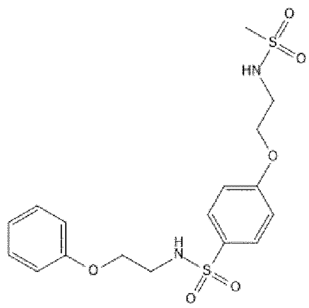 |
| C29 | Cn1c(CNC(=O)c2cccs2)nnc1SCC(=O)Nc1ccc(Cl)cc1 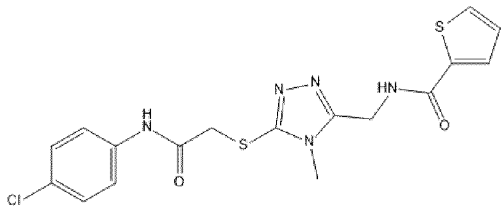 |
| C30 | CNC(=O)NC(=O)CSc1nc2cc(-c3ccc(F)cc3)sc2c(=O)n1N 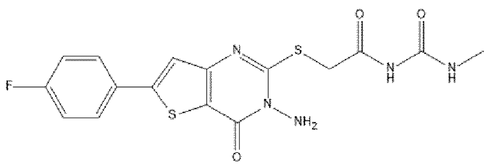 |

C13 in Table 2 is the compound A1, and C14 is the compound A2.

Further referring to FIG. 7, after the part 31 of simulated drug molecule acquisition and the part 32 of virtual screening of simulated drug molecule, there is the part 33 of viability test on screened target drug small molecule (Table 2). The following describes the embodiment of viability test on the screened target drug small molecule.

For example, further referring to FIG. 6, in step S240, a cell viability test is performed on the target drug small molecule to obtain an antagonist of the target receptor molecule.

The target drug small molecules C1-C30 are obtained by outsourcing or synthesis for the following viability test.

In an exemplary embodiment, when the target receptor molecule is a human glucagon receptor, FIG. 13 shows a specific implementation of performing a cell viability test on the target drug small molecule.

Step S910. Select an antagonist drug of hGCGR as a positive control, and mix each target drug small molecule, a glucagon peptide segment, and human embryonic kidney cells containing the target receptor molecule. The glucagon

peptide segment binds to hGCGR to activate the hGCGR signaling pathway and release the second messenger cAMP. The target drug molecule acts as an antagonist to inhibit the activity of hGCGR.

In an exemplary embodiment, an antagonist drug (such as L-168,049) of hGCGR is selected as a positive control. In addition, in view of the human glucagon receptor on the cell membrane of human embryonic kidney cells (such as HEK293), HEK293 may be used to test the drug activity of the human glucagon antagonist. The target drug small molecule, the glucagon peptide segment, and the HEK293 cells containing the target receptor molecule are mixed. The glucagon peptide segment is used to bind to hGCGR to activate the hGCGR signaling pathway and promote the release of the second messenger cAMP. The target drug molecule acts as an antagonist to inhibit the activity of hGCGR.

Step S920. Detect the second messenger cAMP through HTRF, and determine a half maximal inhibitory concentration value of each target drug small molecule according to an intensity value of HTRF.

In an exemplary embodiment, the activity of each target drug small molecule (such as 30 target drug small molecules shown in Table 2) is tested by using the HTRF technique. Specifically, after activation of the glucagon receptor by the glucagon peptide segment, the second messenger cAMP is released from the glucagon receptor cells, the second messenger cAMP may be detected by HTRF, and the half maximal inhibitory concentration (IC50) of the drug molecule may be calculated according to the fluorescence intensity.

Step S930. Determine an antagonist of the target receptor molecule from the target drug small molecules according to the half maximal inhibitory concentration value.

In an exemplary embodiment, for the 30 target drug small molecules shown in Table 2, 11 identical concentration gradients are set for each target drug small molecule. The activity of each target drug small molecule is determined by the HTRF second messenger cAMP response experiment in triplicate. The experimental data obtained is shown in Table 3.

Table 3

| | C1 | | | C2 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -5 | -28.0258 | -21.233 | -13.007 | 3.236993 | 24.94373 | 13.36669 | 100.8895 |
| -5.477121 | -14.4564 | -9.71743 | -14.5956 | 1.776627 | 7.94289 | -25.2765 | 98.1315 |
| -5.954243 | 15.2744 | -5.04528 | 24.07916 | -15.0817 | -15.3237 | 4.391063 | 94.46029 |
| -6.431364 | 9.37852 | -4.14799 | 14.31144 | -0.53168 | -15.9398 | -32.6918 | 88.0059 |
| -6.908485 | -18.9021 | 21.35055 | 4.698388 | -18.7178 | -9.89474 | -3.86237 | 74.79468 |
| -7.385606 | -25.9558 | 3.252519 | -8.77172 | -6.16686 | 15.0145 | -21.4454 | 16.30664 |
| -7.862728 | 17.54573 | -3.01577 | -15.9679 | 9.494435 | -19.3489 | -6.35426 | 15.3532 |
| -8.339849 | 23.35759 | 11.77735 | 18.98269 | -16.8681 | 6.175196 | -11.2756 | -9.20905 |
| -8.81697 | 3.261729 | -32.569 | -19.2486 | 3.217721 | -18.2214 | -23.1148 | -3.66343 |
| -9.294091 | -23.3919 | -4.769 | -10.8733 | 1.325076 | 0.80065 | 5.406518 | -1.83995 |
| -9.771213 | 2.845776 | -32.6062 | -21.4687 | -13.0928 | -15.3836 | -32.7196 | 6.132259 |
| | C3 | | | C4 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 17.5365 | -19.8571 | 13.07843 | -7.98023 | 18.30761 | 8.109144 | 100.8895 |
| -5.477121 | 7.062952 | -13.1408 | -20.3045 | -21.977 | 3.450386 | -34.0441 | 98.1315 |
| -5.954243 | -27.5202 | -34.2999 | 8.031528 | 23.03118 | 21.855 | -35.0246 | 94.46029 |
| -6.431364 | -14.7068 | 8.213836 | -19.4572 | -17.516 | 29.34127 | 0.099727 | 88.0059 |
| -6.908485 | 5.050306 | 4.704503 | 12.01524 | -34.2076 | -17.0491 | 5.095997 | 74.79468 |
| -7.385606 | -11.469 | -14.3269 | -26.5832 | -10.2779 | -43.5579 | -44.3127 | 16.30664 |
| -7.862728 | -4.82247 | 22.19872 | 37.22665 | -22.7041 | 13.11766 | 14.0343 | 15.3532 |
| -8.339849 | 38.45775 | -11.5852 | 6.377197 | -39.6923 | -37.7534 | -28.0946 | -9.20905 |
| -8.81697 | -37.1112 | 24.86409 | 25.39576 | -29.3017 | 28.12523 | -35.2389 | -3.66343 |
| -9.294091 | -12.3879 | 22.72449 | -24.7055 | -22.7752 | -7.6478 | 10.39849 | -1.83995 |

(continued)

| | C3 | | | C4 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -9.771213 | -7.94458 | -38.3808 | -38.8089 | 8.16323 | -15.6864 | -11.0532 | 6.132259 |
| | C5 | | | C6 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | -4.47669 | 2.99794 | 10.59306 | 1.324715 | -19.4427 | -0.59812 | 100.8895 |
| -5.477121 | -8.81744 | 9.731563 | 24.85593 | -7.39909 | -31.1356 | 11.61118 | 98.1315 |
| -5.954243 | -19.0769 | 16.9306 | 32.16685 | 2.828327 | -16.4923 | -14.7246 | 94.46029 |
| -6.431364 | -40.8977 | 36.99523 | 19.7015 | -1.20885 | -33.8383 | -27.1264 | 88.0059 |
| -6.908485 | -11.8532 | 21.80451 | -9.38534 | 22.74271 | -29.6222 | -13.735 | 74.79468 |
| -7.385606 | 2.139664 | -31.8124 | 14.4573 | -10.0239 | 7.609363 | -37.6003 | 16.30664 |
| -7.862728 | 13.23674 | 0.887792 | -7.86726 | -28.7792 | -19.4453 | -24.9236 | 15.3532 |
| -8.339849 | -13.3723 | 0.993211 | 27.5293 | -21.4961 | 2.644305 | -14.7628 | -9.20905 |
| -8.81697 | 20.063 | 2.106405 | 7.734295 | 24.56327 | -7.31118 | -4.04869 | -3.66343 |
| -9.294091 | 5.655235 | 15.5193 | -16.5791 | -8.6768 | -2.92442 | -18.0637 | -1.83995 |
| -9.771213 | 4.21249 | -20.4926 | 12.41512 | -17.3174 | -6.7556 | -19.5629 | 6.132259 |
| | C7 | | | C8 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 24.68995 | -1.7966 | -19.0029 | -2.87803 | -5.23341 | -26.9314 | 100.8895 |
| -5.477121 | -10.0731 | -17.914 | -39.5885 | -18.766 | -3.18983 | -10.0851 | 98.1315 |
| -5.954243 | -14.3481 | 13.8798 | -23.9423 | -26.881 | -25.9253 | 29.23846 | 94.46029 |
| -6.431364 | -7.03772 | 17.46511 | 8.593303 | -11.2606 | -15.2183 | -22.7106 | 88.0059 |
| -6.908485 | 0.252328 | -26.289 | -16.2536 | -38.3508 | 24.06692 | 0.862923 | 74.79468 |
| -7.385606 | -7.9042 | 9.754791 | -36.828 | -37.3765 | 18.66184 | 30.04996 | 16.30664 |

(continued)

| | C7 | | | C8 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -7.862728 | -25.3779 | -25.5195 | -24.1306 | -27.7846 | 12.04921 | -9.82414 | 15.3532 |
| -8.339849 | 10.94666 | -18.4292 | -40.1562 | -31.861 | 22.59342 | -1.72845 | -9.20905 |
| -8.81697 | -3.84146 | -1.60828 | 16.50199 | -39.1634 | -28.8935 | -40.0203 | -3.66343 |
| -9.294091 | -8.84657 | -15.2446 | -18.5536 | 7.386567 | 25.68153 | -17.1855 | -1.83995 |
| -9.771213 | -4.61808 | -34.3714 | 18.07751 | -20.3263 | -18.7347 | 11.37383 | 6.132259 |
| | C9 | | | C10 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 4.489008 | 5.780424 | -12.3845 | 32.72604 | -1.3151 | 6.509968 | 100.8895 |
| -5.477121 | 11.7879 | -28.9568 | 40.0395 | -40.5518 | -33.9801 | -29.38 | 98.1315 |
| -5.954243 | -27.4365 | -18.8961 | -2.50749 | 1.691881 | 1.059233 | 6.714616 | 94.46029 |
| -6.431364 | -0.31304 | -39.3941 | 8.568971 | 5.142818 | 22.78571 | 17.93225 | 88.0059 |
| -6.908485 | -5.30467 | 29.2878 | 1.821058 | -33.073 | -31.3707 | -29.8413 | 74.79468 |
| -7.385606 | 3.734113 | -2.05009 | -2.9843 | -10.1816 | -6.75334 | 26.24217 | 16.30664 |
| -7.862728 | -39.2155 | -18.1272 | -20.2487 | 15.12705 | -1.73848 | 6.046685 | 15.3532 |
| -8.339849 | -0.12562 | -13.9689 | -7.52782 | 8.978043 | -22.8617 | -7.75883 | -9.20905 |
| -8.81697 | 8.520622 | 5.537094 | 14.33727 | -2.4201 | -9.22566 | -27.2736 | -3.66343 |
| -9.294091 | -8.9486 | 1.419335 | -17.5352 | 1.539957 | 4.023809 | -27.1512 | -1.83995 |
| -9.771213 | 11.24507 | 24.89889 | -7.66726 | 37.53523 | -6.39515 | -0.95943 | 6.132259 |
| | C11 | | | C12 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 21.45849 | 27.00526 | 27.99146 | 4.799508 | 9.81866 | 33.12021 | 100.4281 |
| -5.477121 | -31.6507 | 7.262713 | -22.9178 | 17.07896 | 10.71564 | -24.3861 | 98.6132 |

(continued)

| | C11 | | | C12 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -5.954243 | -4.23774 | -36.7341 | 20.91248 | -6.27828 | -0.39781 | -34.2094 | 98.05019 |
| -6.431364 | -5.12705 | 20.93564 | 1.209367 | -19.6532 | 18.26483 | 23.78456 | 93.04975 |
| -6.908485 | -2.04142 | 19.82091 | -11.6525 | -12.7604 | -44.4926 | -26.034 | 89.45811 |
| -7.385606 | 23.70626 | 17.97183 | 5.904263 | 34.20857 | 34.3645 | -6.3512 | 47.8261 |
| -7.862728 | 6.249828 | -9.98796 | 0.828934 | -15.5049 | -21.1087 | -35.3885 | 28.98286 |
| -8.339849 | 11.37375 | 16.24813 | 12.51833 | -22.9805 | -5.84707 | -6.17003 | 3.941144 |
| -8.81697 | -7.93664 | -6.3406 | 10.11275 | 12.37748 | -1.73804 | 20.01369 | -8.05055 |
| -9.294091 | -26.4146 | -18.261 | -33.6664 | 3.022523 | -13.8837 | 0.217719 | -3.76273 |
| -9.771213 | -20.9598 | 11.81976 | -13.6145 | 18.0501 | -20.103 | -38.0781 | -1.911 |
| | C13 | | | C14 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 98.36784 | 95.2799 | 96.14817 | 103.2945 | 102.1097 | 103.2736 | 100.4281 |
| -5.477121 | 87.84198 | 83.27731 | 83.50867 | 106.1285 | 105.9119 | 107.3553 | 98.6132 |
| -5.954243 | 75.44346 | 83.23603 | 79.30265 | 84.31701 | 92.47757 | 91.04928 | 98.05019 |
| -6.431364 | 54.65901 | 24.56393 | 41.98704 | 40.16475 | 62.73851 | 47.64906 | 93.04975 |
| -6.908485 | -13.6386 | 2.206529 | -0.67812 | 42.6475 | 14.48349 | 29.53025 | 89.45811 |
| -7.385606 | 35.8205 | 18.90853 | 45.6947 | 5.293268 | 31.48621 | 31.78721 | 47.8261 |
| -7.862728 | 7.61974 | -2.13549 | -0.78096 | -24.902 | 51.04536 | 18.00119 | 28.98286 |
| -8.339849 | 30.02611 | 27.22016 | 3.938939 | 33.24314 | -0.7378 | 10.67377 | 3.941144 |
| -8.81697 | -22.4972 | 19.34481 | 15.31759 | -19.9622 | 10.71795 | 11.92987 | -8.05055 |
| -9.294091 | 4.979932 | -28.5437 | 23.09991 | 15.16754 | 29.87882 | 11.08606 | -3.76273 |
| -9.771213 | 3.18964 | -35.8803 | -3.23056 | -4.69973 | 3.389463 | -14.8291 | -1.911 |

(continued)

| | C15 | | | C16 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -5 | 2.450829 | 0.025709 | 3.528513 | 9.049217 | 12.23356 | -4.64098 | 100.4281 |
| -5.477121 | -27.0586 | 9.931496 | 22.98634 | -39.9404 | 6.381835 | -17.9195 | 98.6132 |
| -5.954243 | 7.391346 | -32.1137 | -21.9369 | -11.9388 | 15.0488 | -17.1213 | 98.05019 |
| -6.431364 | -27.031 | -17.0364 | -35.2534 | -10.0734 | 23.95272 | 14.02808 | 93.04975 |
| -6.908485 | -19.189 | -13.1875 | -18.6652 | -5.22099 | -7.47664 | -6.35807 | 89.45811 |
| -7.385606 | -1.53526 | -25.3583 | -1.38134 | -34.3838 | -5.34739 | -17.0011 | 47.8261 |
| -7.862728 | 3.911324 | 12.70592 | 11.15824 | 29.49345 | -35.6517 | -4.33241 | 28.98286 |
| -8.339849 | -1.82505 | -0.32445 | 22.70625 | 0.113784 | -22.168 | 2.692058 | 3.941144 |
| -8.81697 | 8.351451 | 1.505195 | 30.16285 | -7.7562 | -6.83617 | -15.6271 | -8.05055 |
| -9.294091 | -22.3536 | -0.35562 | 18.35494 | -18.7476 | -0.88687 | -4.2237 | -3.76273 |
| -9.771213 | -29.5339 | -3.45996 | 2.160174 | -11.5867 | -27.0158 | -2.25023 | -1.911 |
| | C17 | | | C18 | | | L-168,049 |
| Concentration gradient LogM | Inhibition percentage | Inhibition percentage | Inhibition percentage | Inhibition percentage | Inhibition percentage | Inhibition percentage | Inhibition percentage |
| | | | | | | | |
| -5 | 7.814834 | -18.7271 | -39.712 | 20.41717 | -21.9235 | 39.73831 | 100.4281 |
| -5.477121 | -21.4793 | 21.66169 | -34.5604 | -47.4694 | -7.05033 | -26.1305 | 98.6132 |
| -5.954243 | -43.3089 | 0.113663 | -18.0925 | -6.59348 | -12.6979 | -7.93561 | 98.05019 |
| -6.431364 | -23.0497 | -5.46763 | -15.6262 | -17.6346 | -19.0398 | -30.7091 | 93.04975 |
| -6.908485 | -24.7454 | -43.349 | -22.0993 | -1.7783 | 1.234805 | -33.0095 | 89.45811 |
| -7.385606 | 7.80625 | -39.1892 | -15.7379 | -23.0256 | -11.8751 | 28.0322 | 47.8261 |
| -7.862728 | -7.46562 | -68.4482 | -20.2046 | 12.90464 | -9.84093 | -11.508 | 28.98286 |
| -8.339849 | -22.1581 | 13.25272 | -27.493 | -32.3776 | 1.360505 | -14.8079 | 3.941144 |
| -8.81697 | 10.6136 | 2.858905 | -44.4852 | 32.81712 | 28.79609 | -38.9051 | -8.05055 |

(continued)

| | C17 | | | C18 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| | | | | | | | |
| -9.294091 | 3.42252 | 8.940037 | -51.9949 | 9.372862 | -12.2856 | -13.1795 | -3.76273 |
| -9.771213 | 1.878418 | 11.84952 | -9.81694 | -5.78329 | -6.94506 | 15.9268 | -1.911 |
| | C19 | | | C20 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 34.07259 | -35.2218 | -6.71092 | 18.1581 | -39.4147 | 2.043708 | 100.4281 |
| -5.477121 | 11.30968 | 29.49002 | 31.79587 | -16.9934 | 2.365699 | -3.05239 | 98.6132 |
| -5.954243 | 10.99531 | 5.631229 | 41.03634 | 12.90692 | -13.0114 | -8.73296 | 98.05019 |
| -6.431364 | 18.95094 | 10.74806 | -13.2223 | 7.253805 | -8.40831 | 5.904631 | 93.04975 |
| -6.908485 | -25.5981 | 0.985976 | 0.434258 | 24.47322 | 14.07576 | 33.94305 | 89.45811 |
| -7.385606 | -1.71538 | 24.36076 | -4.49796 | -9.86884 | 2.657783 | 34.57254 | 47.8261 |
| -7.862728 | 6.139602 | 14.66942 | -12.5289 | -8.83545 | 12.40652 | -24.3369 | 28.98286 |
| -8.339849 | -35.1624 | -10.5336 | -2.96128 | 3.4469 | -8.56771 | -5.41089 | 3.941144 |
| -8.81697 | 31.1282 | -1.14472 | -11.2501 | 14.95257 | 16.5898 | 44.82866 | -8.05055 |
| -9.294091 | 10.09647 | 28.37046 | 17.52129 | -40.3815 | 9.302105 | 9.666315 | -3.76273 |
| -9.771213 | -6.75399 | -19.8704 | 11.16038 | -1.23546 | 5.313066 | 21.17244 | -1.911 |
| | C21 | | | C22 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | -42.7197 | -40.82 | -6.17334 | -30.5862 | 16.50023 | -1.00033 | 100.5604 |
| -5.477121 | -53.9423 | -47.186 | -21.3641 | -57.5005 | -12.8689 | -34.1965 | 96.62153 |
| -5.954243 | 21.78096 | -1.82111 | 3.319337 | -11.4422 | 17.90716 | -23.1384 | 95.17342 |
| -6.431364 | 27.77292 | -18.0508 | 9.924084 | 65.059200* | -9.83757 | -38.5336 | 91.89666 |

(continued)

| | C21 | | | C22 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -6.908485 | 55.969900* | 67.355300* | -14.3303 | 59.258300* | -48.0506 | -31.7455 | 78.16119 |
| -7.385606 | -38.8149 | -0.62746 | -54.5274 | -5.73492 | 1.577532 | -3.87531 | 46.68825 |
| -7.862728 | -19.5624 | 26.74542 | -38.8977 | -41.331 | -24.4309 | 23.83835 | 33.52469 |
| -8.339849 | -16.4657 | -37.8127 | 25.10431 | -3.2511 | 21.51812 | -22.2925 | -19.5336 |
| -8.81697 | -28.5528 | 13.99508 | -19.2725 | -1.04743 | -27.6336 | -52.9651 | 22.63393 |
| -9.294091 | 33.24786 | -16.9023 | 17.91219 | -5.112 | 10.46791 | -17.7298 | 19.0859 |
| -9.771213 | -49.0707 | -18.6164 | -5.68068 | -34.2012 | 3.477508 | -5.28374 | 1.646174 |
| | C23 | | | C24 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 75.66265 | 75.13544 | 57.57891 | -10.9856 | 2.297213 | -15.1187 | 100.5604 |
| -5.477121 | 20.07258 | 43.03023 | 5.134522 | -6.38747 | -23.2283 | 7.8078 | 96.62153 |
| -5.954243 | -0.8394 | 41.49496 | 8.766312 | -12.0514 | 0.761549 | 1.112782 | 95.17342 |
| -6.431364 | -15.2381 | -9.68248 | 6.049052 | -49.0215 | -15.3222 | -46.9302 | 91.89666 |
| -6.908485 | 4.280152 | 12.02751 | 71.091500* | -52.9455 | -56.6235 | -5.83182 | 78.16119 |
| -7.385606 | -31.8529 | 12.3235 | -11.7931 | -6.07678 | -5.63608 | -26.4358 | 46.68825 |
| -7.862728 | 76.091900* | -26.5916 | -31.2095 | -5.35373 | -21.4306 | -33.6957 | 33.52469 |
| -8.339849 | -15.8448 | 23.02911 | -17.3448 | -42.5342 | -2.30491 | -36.5898 | -19.5336 |
| -8.81697 | -28.8951 | 17.21836 | 14.27307 | -23.6956 | 17.50362 | -36.9708 | 22.63393 |
| -9.294091 | 7.800055 | 24.08749 | -12.9822 | -16.2822 | 0.171539 | -55.6223 | 19.0859 |
| -9.771213 | -42.3231 | 44.27058 | -2.62025 | -66.6615 | -3.7793 | -50.5947 | 1.646174 |

(continued)

| Concentration gradient LogM | C25 | | | C26 | | | L-168,049 |
|---|---|---|---|---|---|---|---|
| | Inhibition percentage | Inhibition percentage | Inhibition percentage | Inhibition percentage | Inhibition percentage | Inhibition percentage | Inhibition percentage |
| -5 | 8.161618 | -0.73687 | 49.3189 | 16.60552 | -26.5175 | -5.9826 | 100.5604 |
| -5.477121 | -14.9937 | -20.5786 | 7.536136 | -27.6759 | -0.86548 | -35.8233 | 96.62153 |
| -5.954243 | -31.4725 | 1.59208 | -42.8834 | -39.1019 | -16.9961 | -11.7971 | 95.17342 |
| -6.431364 | 27.05132 | 7.403896 | 41.96233 | -26.4461 | -52.8129 | -39.6412 | 91.89666 |
| -6.908485 | -43.5173 | -14.2199 | -76.6858 | -25.9687 | -12.3017 | -11.3498 | 78.16119 |
| -7.385606 | -24.7275 | -36.5786 | 13.76706 | -38.8293 | -45.2629 | 0.210664 | 46.68825 |
| -7.862728 | 17.73872 | 36.43395 | 29.72938 | -14.0435 | -42.9647 | -5.60207 | 33.52469 |
| -8.339849 | -22.3882 | -39.6366 | -40.6625 | 5.791446 | -19.5966 | 9.198763 | -19.5336 |
| -8.81697 | -32.0254 | -37.1231 | -52.6473 | -9.84122 | -10.6246 | -47.3524 | 22.63393 |
| -9.294091 | -56.6628 | -19.4824 | 10.50452 | -0.1124 | 9.630004 | 28.05575 | 19.0859 |
| -9.771213 | -140.839 | -8.89688 | 17.2997 | -17.8823 | 2.340118 | 20.41762 | 1.646174 |
| | C27 | | | C28 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 17.73558 | -48.9678 | 36.75353 | 31.40869 | -54.3544 | -60.6171 | 100.5604 |
| -5.477121 | -13.4598 | -78.7253 | 6.21246 | 14.39916 | 19.28817 | -55.5558 | 96.62153 |
| -5.954243 | -68.7512 | -13.923 | -68.5079 | -24.2875 | 38.00314 | -25.343 | 95.17342 |
| -6.431364 | -29.4768 | -45.8369 | -48.8129 | 34.36428 | -1.71369 | -37.8756 | 91.89666 |
| -6.908485 | -2.49091 | -20.0347 | -51.5915 | -43.4925 | 5.619733 | 2.448334 | 78.16119 |
| -7.385606 | -12.2442 | 25.23883 | -48.6066 | -21.3641 | -7.80414 | -1.89249 | 46.68825 |
| -7.862728 | -12.8566 | -60.2134 | 1.10327 | 14.56813 | 25.73587 | -8.62761 | 33.52469 |
| -8.339849 | -24.7315 | -29.8299 | -20.459 | -6.70335 | -16.2867 | -3.68355 | -19.5336 |
| -8.81697 | -19.8783 | 20.75261 | -6.84695 | -27.3759 | 21.56875 | 34.83851 | 22.63393 |
| -9.294091 | -30.7083 | -13.4815 | -50.8699 | -50.8468 | 11.9284 | -1.61008 | 19.0859 |

(continued)

| | C27 | | | C28 | | | L-168,049 |
|---|---|---|---|---|---|---|---|
| Concentration gradient LogM | Inhibition percentage | Inhibition percentage | Inhibition percentage | Inhibition percentage | Inhibition percentage | Inhibition percentage | Inhibition percentage |
| -9.771213 | -13.3454 | -44.3837 | -24.0621 | -21.813 | -45.1414 | 6.246463 | 1.646174 |
| | C29 | | | C30 | | | L-168,049 |
| Concentration gradient LogM | Inhibition percentage | Inhibition percentage | Inhibition percentage | Inhibition percentage | Inhibition percentage | Inhibition percentage | Inhibition percentage |
| -5 | 21.38173 | -23.7881 | 30.57214 | -31.6544 | -30.6279 | 24.26864 | 100.5604 |
| -5.477121 | 45.01686 | 12.5193 | 22.19625 | -6.69537 | -22.7315 | -38.7442 | 96.62153 |
| -5.954243 | 45.8205 | 2.137398 | 5.001465 | 10.08965 | -15.8104 | -4.7884 | 95.17342 |
| -6.431364 | -18.994 | -12.4118 | -48.7182 | 13.3859 | -77.9226 | 18.45847 | 91.89666 |
| -6.908485 | -12.9208 | 1.518078 | -12.6029 | 21.79603 | -50.2766 | 9.098346 | 78.16119 |
| -7.385606 | -8.36925 | 3.923759 | 16.36132 | -77.3092 | -39.7196 | -16.5711 | 46.68825 |
| -7.862728 | -10.6825 | -9.79216 | -61.623 | 2.209033 | -48.0745 | 19.49895 | 33.52469 |
| -8.339849 | -61.2695 | -33.3603 | -64.963 | -11.1792 | -39.2013 | -24.2246 | -19.5336 |
| -8.81697 | -26.212 | -34.661 | 4.968303 | -6.91135 | -15.7719 | -47.0794 | 22.63393 |
| -9.294091 | -12.0267 | 1.83427 | -62.0128 | -7.167 | 41.78717 | -53.1934 | 19.0859 |
| -9.771213 | -29.6773 | 7.587787 | -31.3932 | -48.4113 | -5.63768 | 16.31158 | 1.646174 |

The data in Table 3 is processed through software using the concentration gradient as the abscissa and the inhibition percentage as the ordinate, to obtain FIG. 14 and Table 4. This figure shows the inhibition percentages of the responses of L-168,049 and 30 target drug small molecules respectively. L-168,049 is a positive control. It can be learned from this figure that both C13 and C14 have the activity, while the remaining 28 target drug small molecules show no activity value.

Table 4

| Number | IC50(M) | Number | IC50(M) | Number | IC50(M) |
|---|---|---|---|---|---|
| C1 | > 10E-05 | C11 | > 10E-05 | C21 | > 10E-05 |
| C2 | >10E-05 | C12 | >10E-05 | C22 | >10E-05 |
| C3 | >10E-05 | C13 | 4.465E-07 | C23 | >10E-05 |
| C4 | >10E-05 | C14 | 4.225E-07 | C24 | >10E-05 |
| C5 | >10E-05 | C15 | >10E-05 | C25 | >10E-05 |
| C6 | >10E-05 | C16 | >10E-05 | C26 | >10E-05 |
| C7 | >10E-05 | C17 | >10E-05 | C27 | >10E-05 |
| C8 | >10E-05 | C18 | >10E-05 | C28 | >10E-05 |
| C9 | >10E-05 | C19 | >10E-05 | C29 | >10E-05 |
| C10 | >10E-05 | C20 | >10E-05 | C30 | >10E-05 |
| L-168,049 | 6.146E-08 | L-168,049 | 2.082E-08 | L-168,049 | 4.215E-08 |

Table 4 shows the half maximal inhibitory concentration IC50 of 30 tested target drug small molecules. For example, the values of half maximal inhibitory concentration IC50 of compound C13 and compound C14 are small, which are 446.5 nM and 422.5 nM respectively, so they can be used as structurally novel human glucagon receptor antagonists.

This technical solution combines the advantages of deep learning and virtual screening. The simulated drug molecule is obtained through prediction based on a deep learning model, to increase the diversity of the chemical space structure of the simulated drug molecule, so that the skeleton structure of the antagonist determined according to the simulated drug molecule is quite different from the skeleton structure of the existing drug, which is beneficial to improving the diversity of drugs. In addition, the small molecule database to be screened is determined according to the simulated drug molecule, and virtual screening is performed on the small molecule database according to the target receptor molecule, so that the target drug small molecule can directly search for a proper conformation in the protein active pocket of the target receptor, which is beneficial to ensuring the appropriateness of the designed antagonist. It can be seen that this technical solution can reduce the cost and time of drug research and development and is beneficial to improving the diversity of drugs.

Other embodiments of the present disclosure will be apparent to a person skilled in the art from consideration of the specification and practice of the disclosure here. The present disclosure is intended to cover any variation, use, or adaptive change of the present disclosure. These variations, uses, or adaptive changes follow the general principles of the present disclosure and include common general knowledge or common technical means in the art that are not disclosed in the present disclosure. The specification and the embodiments are considered as merely exemplary, and the scope and spirit of the present disclosure are pointed out in the following claims.

It is to be understood that the present disclosure is not limited to the precise structures described above and shown in the accompanying drawings, and various modifications and changes can be made without departing from the scope of the present disclosure.

## Claims

1. A compound, represented by the following structural formula A, or an isomer, metabolite, prodrug, pharmaceutically acceptable ester, or pharmaceutically acceptable salt thereof,

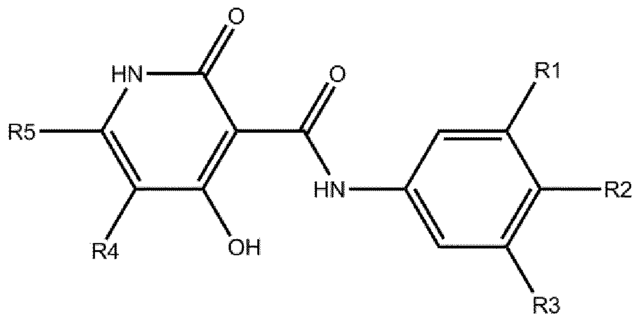

(A),

wherein R1 is H, F, Cl, Br, or I;

R2 is H or $-O-(CH_2)_m-CH_3$, m being an integer of 0-2;
R3 is H or $-S-(CH_2)_n-CH_3$, n being an integer of 0-2; and

R4 and R5 together form

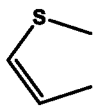

or

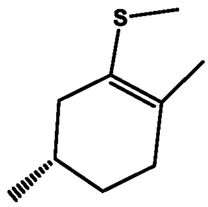

.

2. The compound according to claim 1, wherein in the structural formula A, R1 is -Cl.

3. The compound according to claim 1, wherein in the structural formula A, R2 is $-O-CH_3$.

4. The compound according to claim 1, wherein in the structural formula A, R3 is $-S-CH_3$.

5. The compound according to claim 1, wherein the compound is a compound A1 represented by the following structural formula A1:

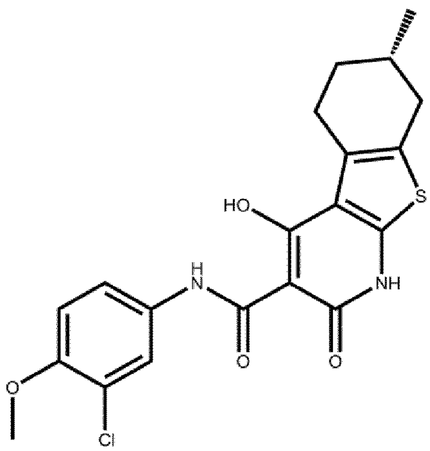

(A1).

6. The compound according to claim 1, wherein the compound is a compound A2 represented by the following structural formula A2:

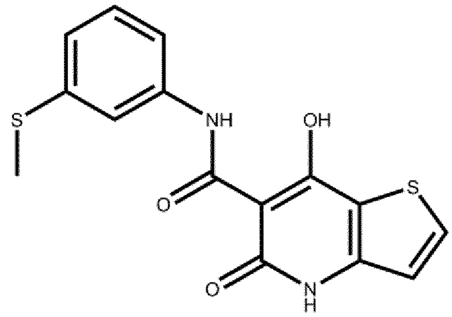

(A2).

7. A pharmaceutical composition, comprising a pharmaceutically acceptable carrier and the compound according to any one of claims 1 to 6.

8. The pharmaceutical composition according to claim 7, wherein the pharmaceutical composition is a solution, a tablet, or a capsule.

9. The pharmaceutical composition according to claim 8, wherein the pharmaceutical composition is administered by injection or orally.

10. Use of the compound according to any one of claims 1 to 6 or the pharmaceutical composition according to any one of claims 7 to 9 in preparing a drug for use as a glucagon receptor antagonist.

11. The use according to claim 10, wherein the drug is used for the treatment of diabetes, disorders of glucagon levels, and/or hyperglycemia.

12. The use according to claim 10, wherein the drug is used for mammals.

13. The use according to claim 12, wherein the drug is used for human.

14. A compound used as a glucagon receptor antagonist, defined by any one of claims 1 to 6.

15. A method for treating diseases associated with dysregulation of glucagon metabolism or regulating blood sugar levels, comprising administering an effective dose of the compound according to any one of claims 1 to 6 or the pharmaceutical composition according to any one of claims 7 to 9 to a subject in need thereof.

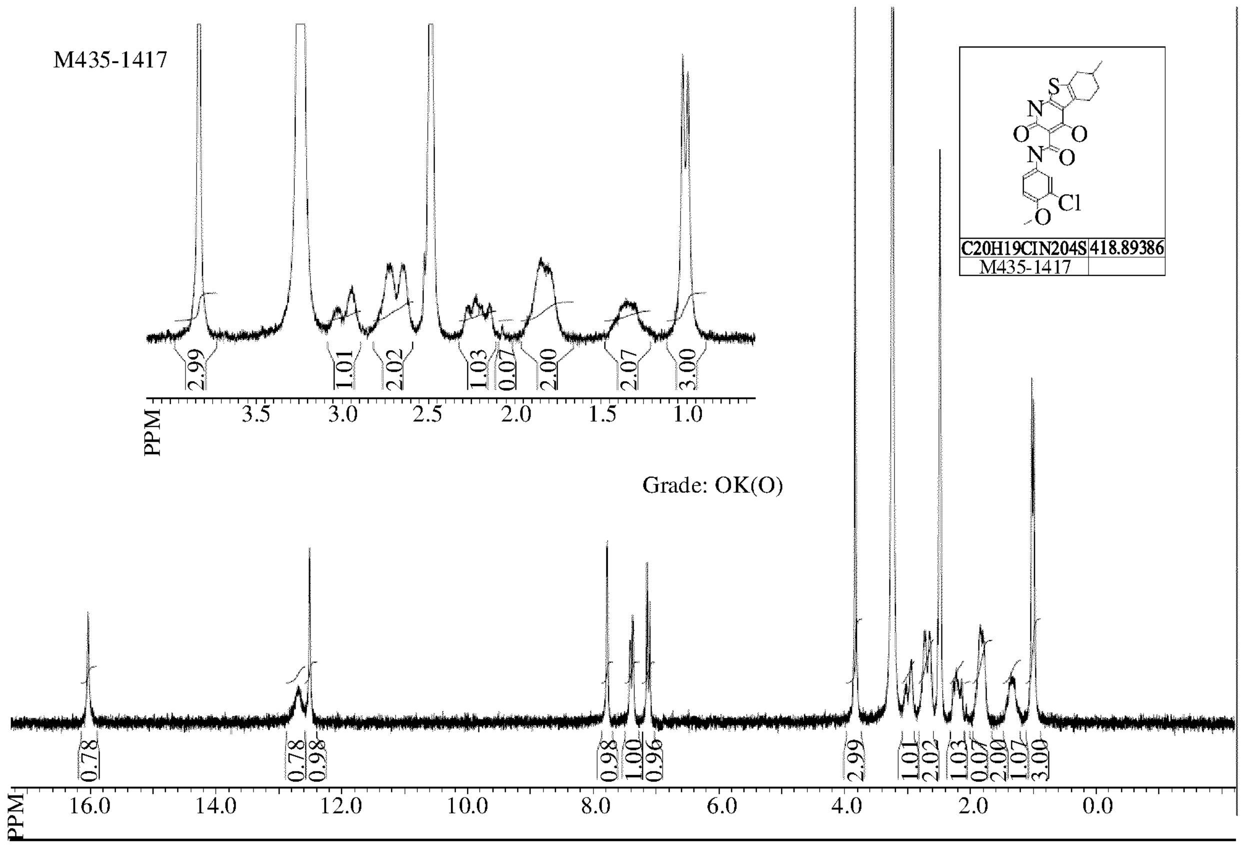
M435-1417
C20H19ClN2O4S 418.89386
M435-1417
Grade: OK(O)
2.99
1.01
2.02
1.03
0.07
2.00
2.07
3.00
PPM
3.5
3.0
2.5
2.0
1.5
1.0
0.78
0.78
0.98
0.98
1.00
0.96
2.99
1.01
2.02
1.03
0.07
2.00
1.07
3.00
PPM
16.0
14.0
12.0
10.0
8.0
6.0
4.0
2.0
0.0

FIG. 1

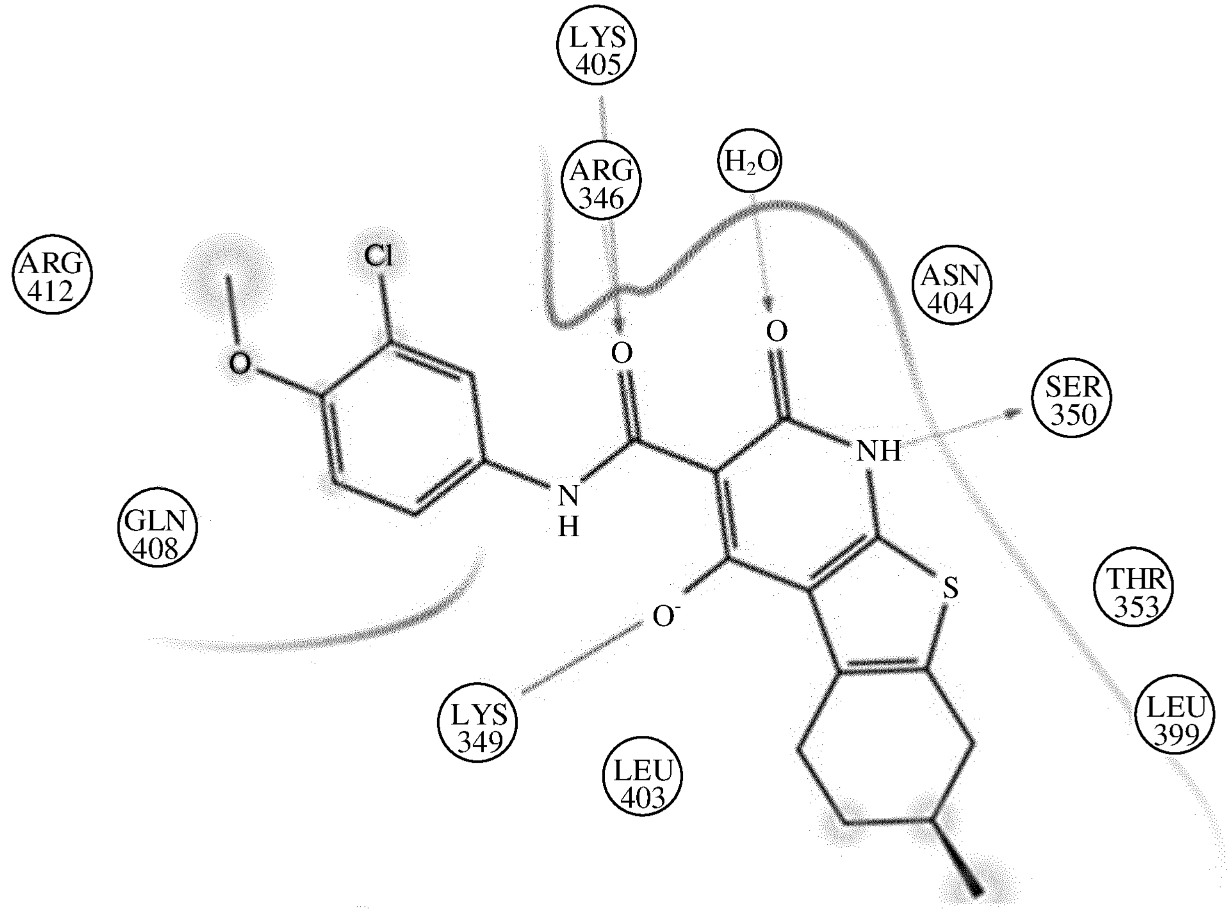

FIG. 2

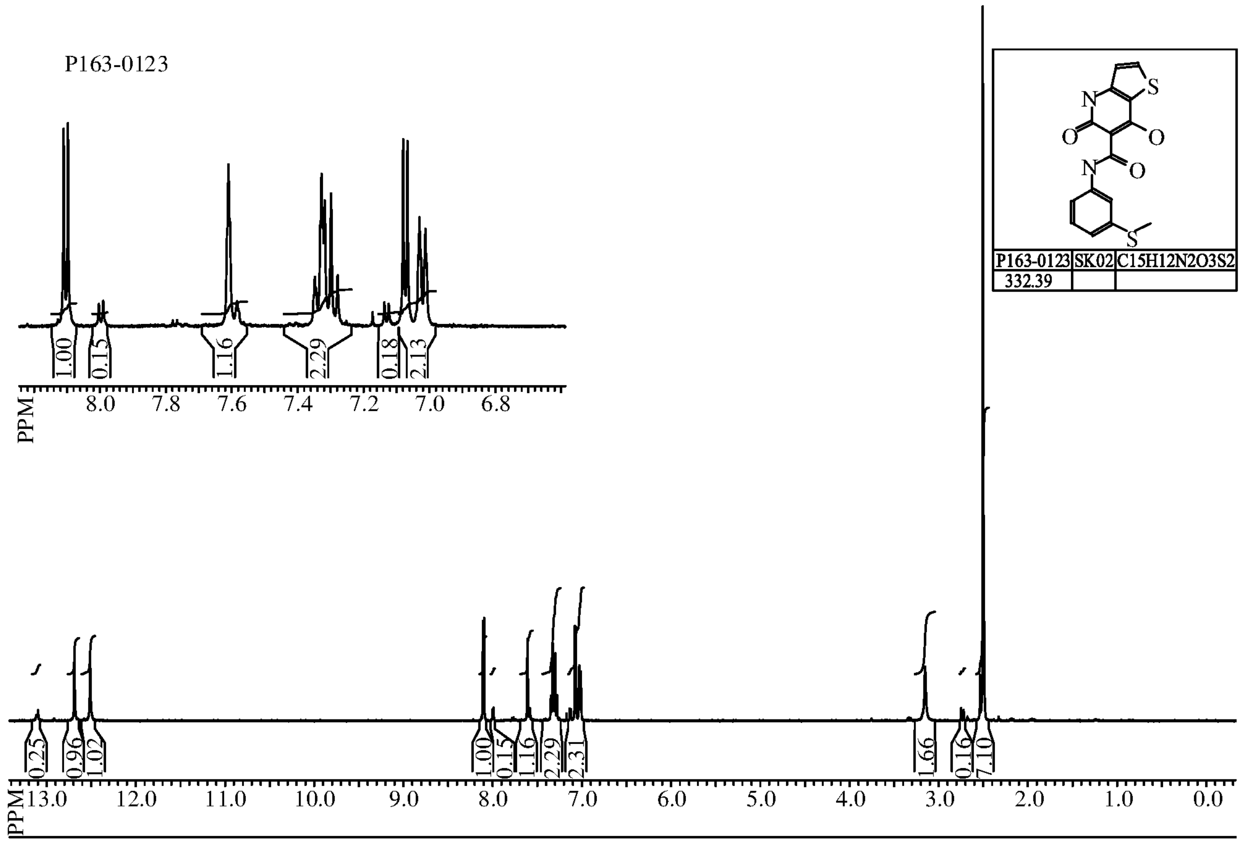
P163-0123
P163-0123 SK02 C15H12N2O3S2
332.39
1.00 0.15 1.16 2.29 0.18 2.13
PPM 8.0 7.8 7.6 7.4 7.2 7.0 6.8
0.25 0.96 1.02 1.00 0.15 1.16 2.29 2.31 1.66 0.16 7.10
PPM 13.0 12.0 11.0 10.0 9.0 8.0 7.0 6.0 5.0 4.0 3.0 2.0 1.0 0.0

FIG. 3

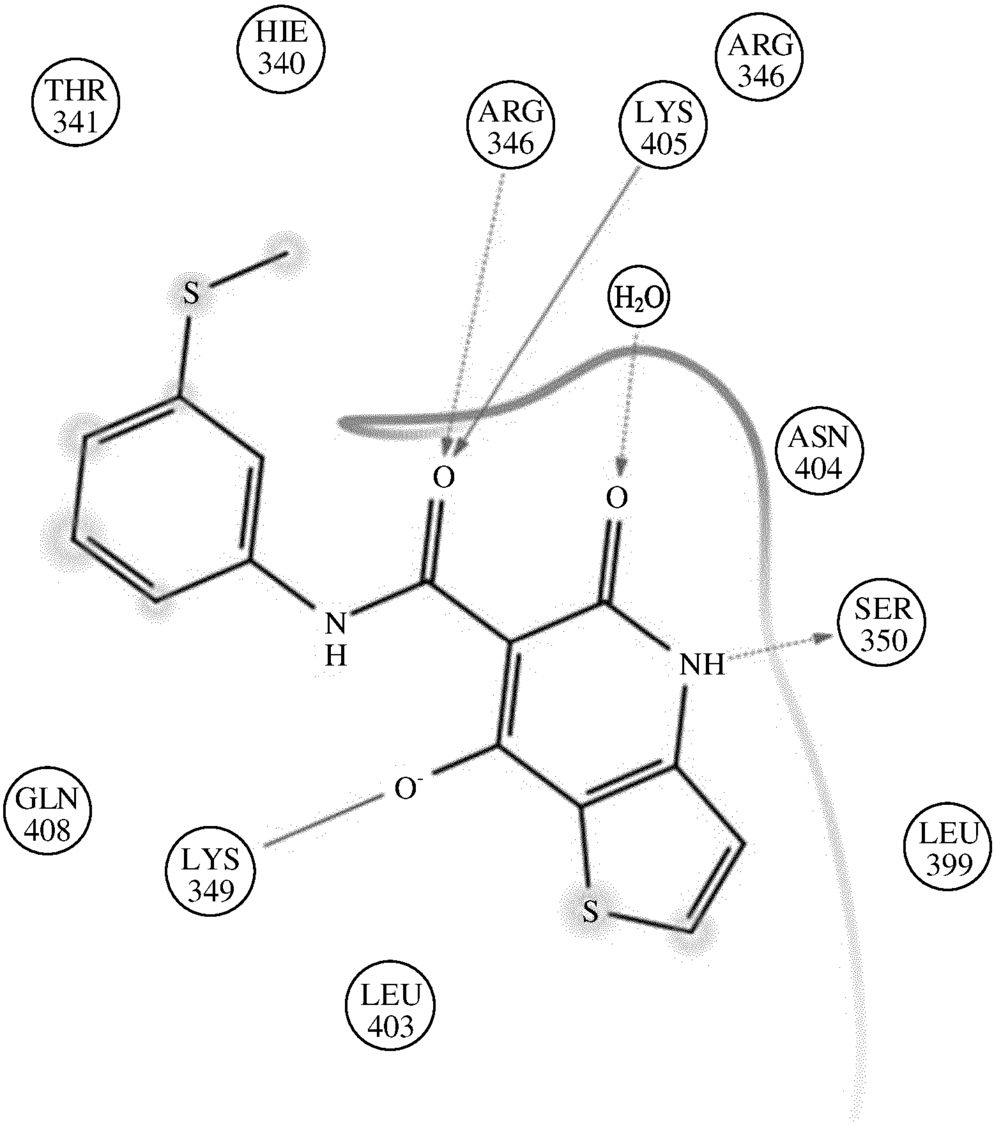

FIG. 4

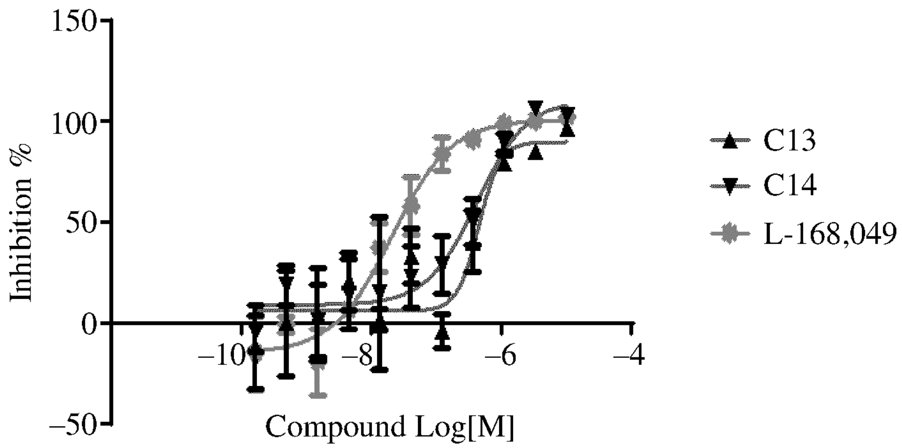
150
100
50
0
−50
Inhibition %
−10
−8
−6
−4
Compound Log[M]
C13
C14
L-168,049

FIG. 5

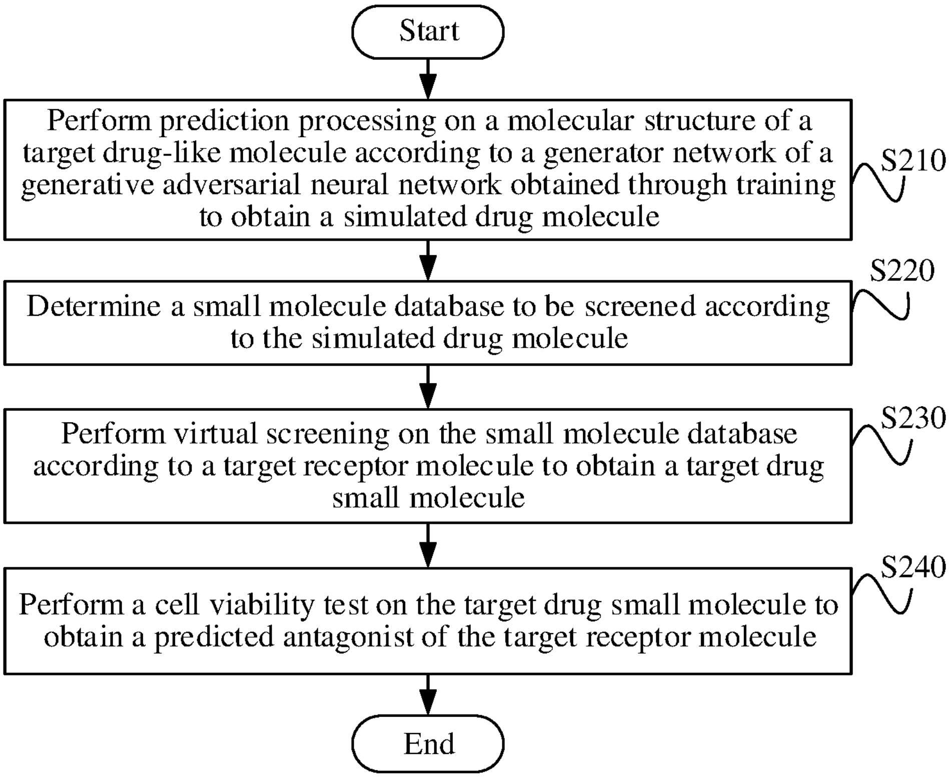
Start
Perform prediction processing on a molecular structure of a target drug-like molecule according to a generator network of a generative adversarial neural network obtained through training to obtain a simulated drug molecule
S210
Determine a small molecule database to be screened according to the simulated drug molecule
S220
Perform virtual screening on the small molecule database according to a target receptor molecule to obtain a target drug small molecule
S230
Perform a cell viability test on the target drug small molecule to obtain a predicted antagonist of the target receptor molecule
S240
End

FIG. 6

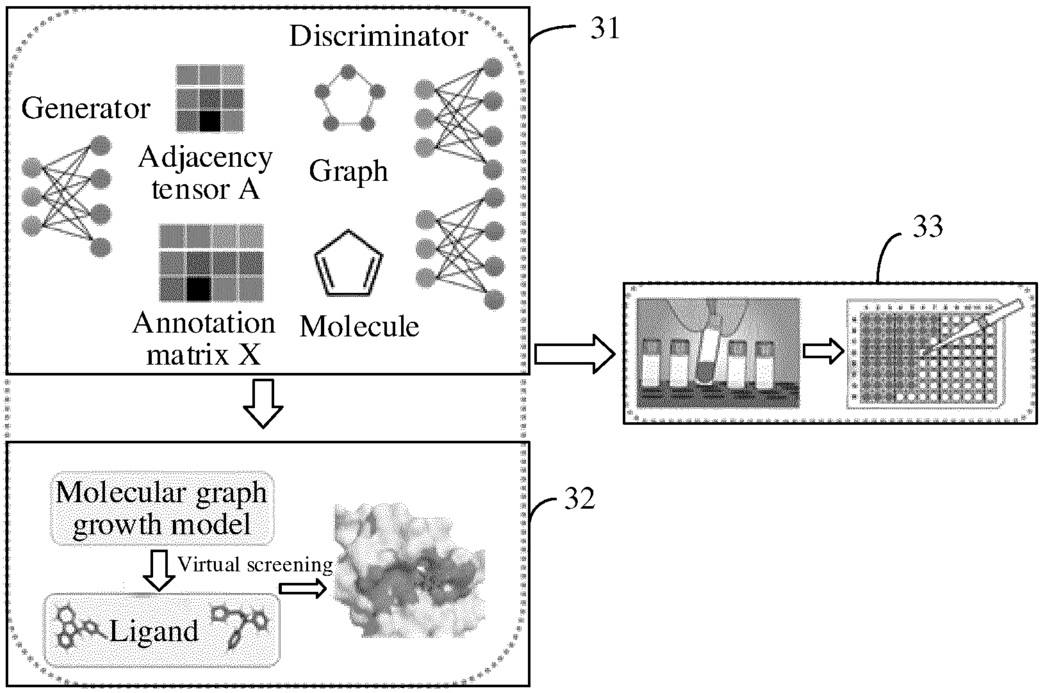
Discriminator
31
Generator
Adjacency tensor A
Graph
Annotation matrix X
Molecule
33
Molecular graph growth model
32
Virtual screening
Ligand

FIG. 7

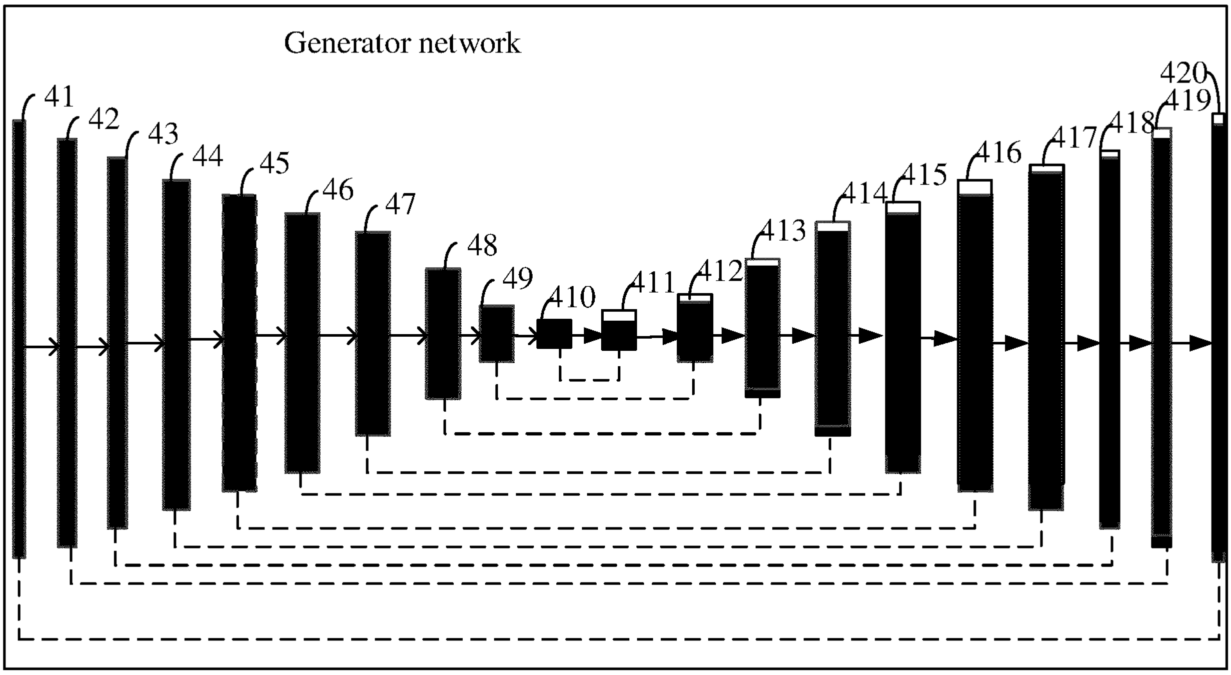
Generator network
41
42
43
44
45
46
47
48
49
410
411
412
413
414
415
416
417
418
419
420

FIG. 8

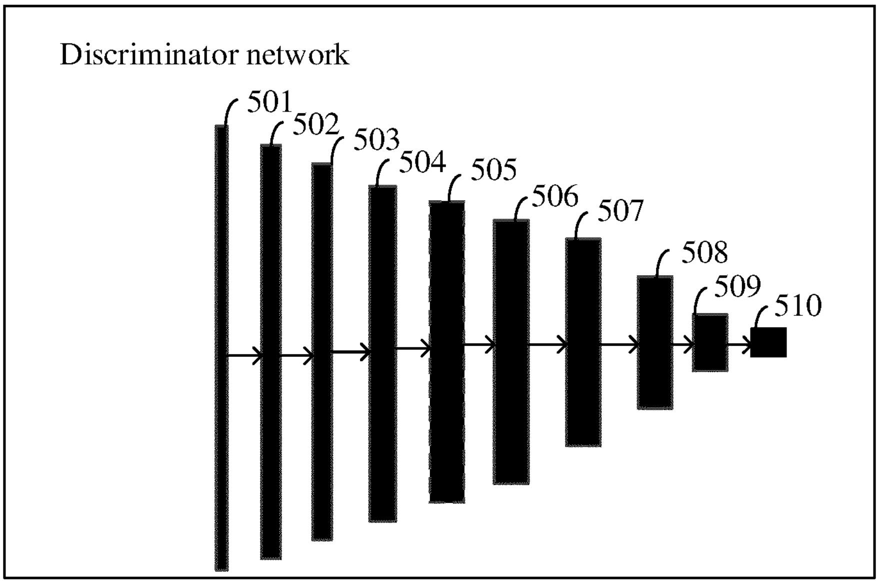
Discriminator network
501
502
503
504
505
506
507
508
509
510

FIG. 9

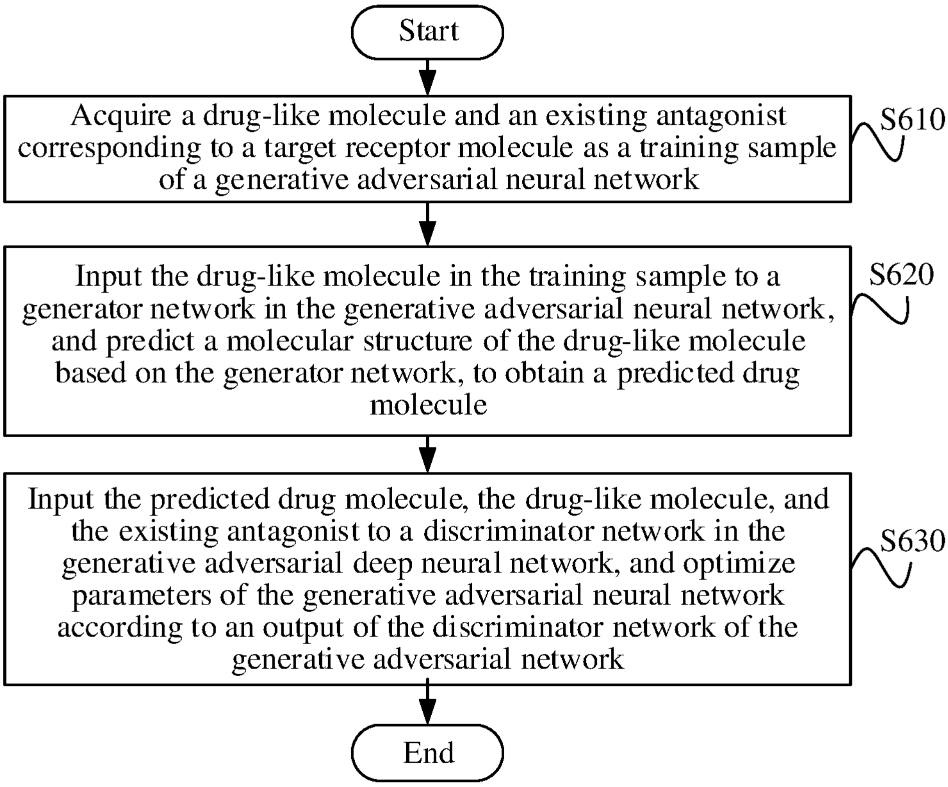


FIG. 10

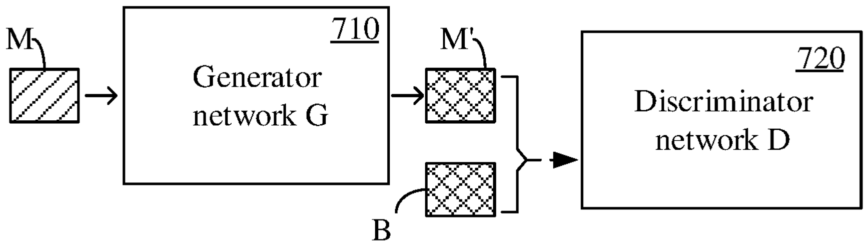


FIG. 11

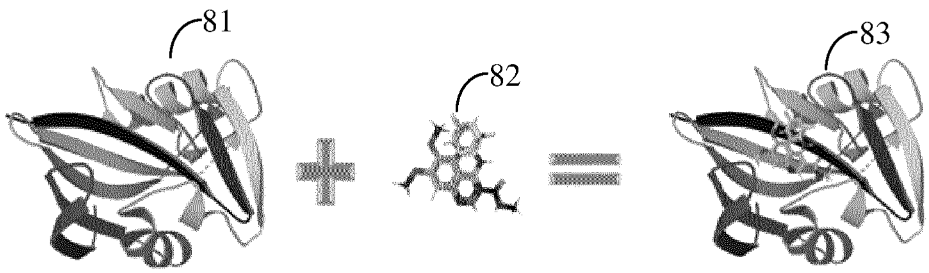


FIG. 12

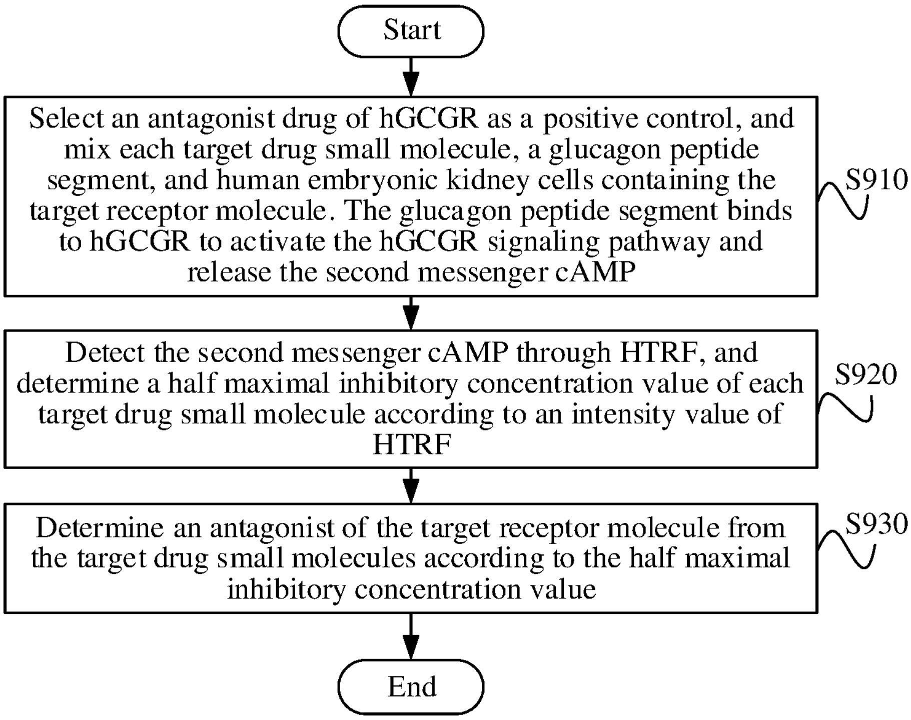
Start
Select an antagonist drug of hGCGR as a positive control, and mix each target drug small molecule, a glucagon peptide segment, and human embryonic kidney cells containing the target receptor molecule. The glucagon peptide segment binds to hGCGR to activate the hGCGR signaling pathway and release the second messenger cAMP
S910
Detect the second messenger cAMP through HTRF, and determine a half maximal inhibitory concentration value of each target drug small molecule according to an intensity value of HTRF
S920
Determine an antagonist of the target receptor molecule from the target drug small molecules according to the half maximal inhibitory concentration value
S930
End

FIG. 13

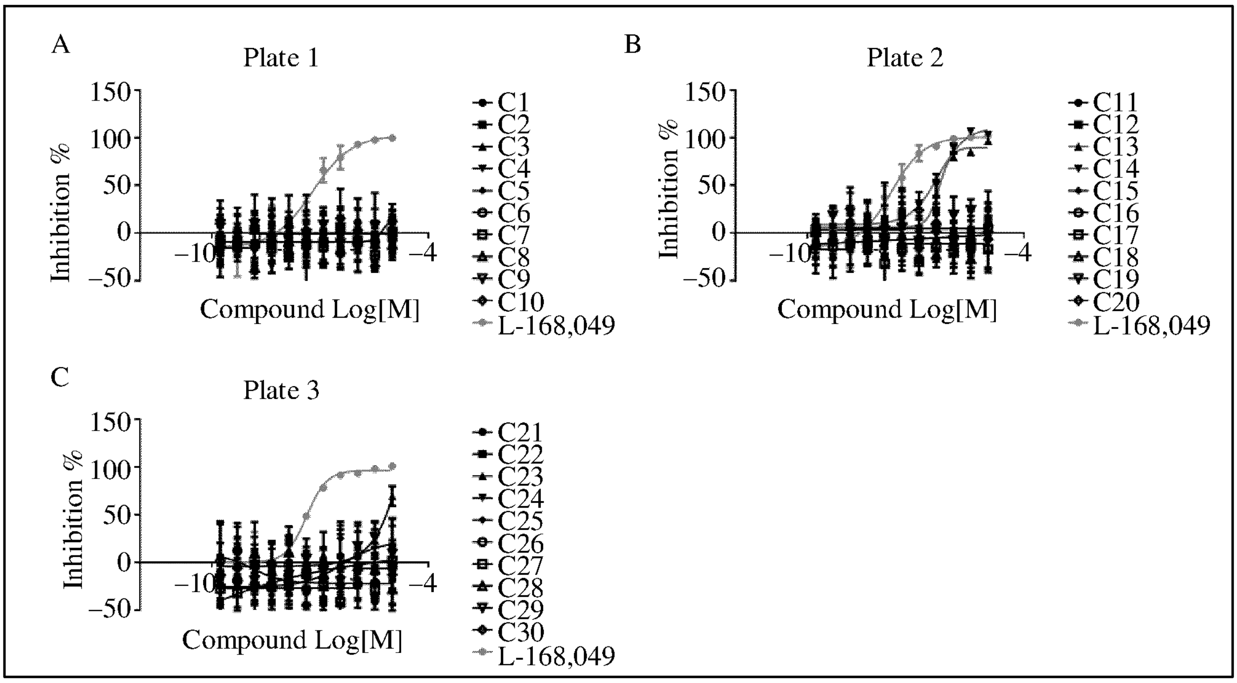
A
Plate 1
Inhibition %
150
100
50
0
−50
−10
−4
Compound Log[M]
C1
C2
C3
C4
C5
C6
C7
C8
C9
C10
L-168,049
B
Plate 2
Inhibition %
Compound Log[M]
C11
C12
C13
C14
C15
C16
C17
C18
C19
C20
L-168,049
C
Plate 3
Inhibition %
Compound Log[M]
C21
C22
C23
C24
C25
C26
C27
C28
C29
C30
L-168,049

FIG. 14

## INTERNATIONAL SEARCH REPORT

International application No.
**PCT/CN2022/076637**

**A. CLASSIFICATION OF SUBJECT MATTER**

C07D 495/04(2006.01)i； A61K 31/4365(2006.01)i； A61P 3/10(2006.01)i

According to International Patent Classification (IPC) or to both national classification and IPC

**B. FIELDS SEARCHED**

Minimum documentation searched (classification system followed by classification symbols)

C07D495/-; A61K31/-; A61P3/-

Documentation searched other than minimum documentation to the extent that such documents are included in the fields searched

Electronic data base consulted during the international search (name of data base and, where practicable, search terms used)

CNTXT; ENTXTC; DWPI; CNKI: 胰高血糖素, 胰高血糖素受体拮抗剂, 高血糖, 降糖, 糖尿病, 环磷酸腺苷, Glucagon, GCGR, glucagon receptor antagonists, hyperglycemia, glucose-lowering agents, diabetes, cyclic adenosine monophosphate, cAMP; REGISTRY(STN), CAPLUS(STN), MARPAT(STN): 基于式(A)进行结构式检索, structural formula search made on the basis of formula (A)

**C. DOCUMENTS CONSIDERED TO BE RELEVANT**

| Category* | Citation of document, with indication, where appropriate, of the relevant passages | Relevant to claim No. |
|---|---|---|
| X | "CAS登记号, CAS Registry No.: 1359641-55-6, 1358514-98-3, 1358514-65-4, 1358349-48-0, 1358349-37-7, 1358349-29-7, 1358202-73-9, 1358202-53-5" *DATABASE REGISTRY(STN),* 02 March 2012 (2012-03-02), | 1-4, 6, 14 |
| A | CN 101495186 A (NOVARTIS AG) 29 July 2009 (2009-07-29) description, pp. 75-76, table 1, and compounds 106-108 and 110-111 | 1-15 |
| A | CN 1774433 A (HOFFMANN LA ROCHE) 17 May 2006 (2006-05-17) description, p. 16, embodiment 6, and claims 23 and 29 | 1-15 |
| A | CN 101146763 A (MERCK & CO. INC.) 19 March 2008 (2008-03-19) entire document | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 April 2022** | **09 May 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/076637**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

[1] Claim 15 relates to a treatment method implemented on a human body or animal body (PCT Rule 39.1(iv)). Nevertheless, a search is still performed on the basis of the technical subject matter of a use of the compound/composition in preparation of a drug for treating diseases associated with dysregulated glucagon metabolism or regulating a blood glucose level.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/076637**

| Patent document cited in search report | Publication date (day/month/year) | Patent family member(s) | Publication date (day/month/year) |
|---|---|---|---|
| CN 101495186 A | 29 July 2009 | WO 2008014307 A2 | 31 January 2008 |
| | | CA 2659605 A1 | 31 January 2008 |
| | | JP 2009544732 A | 17 December 2009 |
| | | MX 2009000943 A | 04 February 2009 |
| | | US 2009325948 A1 | 31 December 2009 |
| | | AU 2007276804 A1 | 31 January 2008 |
| | | KR 20090046872 A | 11 May 2009 |
| | | EP 2059305 A2 | 20 May 2009 |
| CN 1774433 A | 17 May 2006 | EP 1620425 A1 | 01 February 2006 |
| | | AU 2004230220 A1 | 28 October 2004 |
| | | PL 1620425 T3 | 30 June 2009 |
| | | CA 2521732 A1 | 28 October 2004 |
| | | WO 2004092156 A1 | 28 October 2004 |
| | | RU 2005135338 A | 27 May 2007 |
| | | MX PA05010998 A | 12 December 2005 |
| | | BR PI0409454 A | 02 May 2006 |
| | | JP 2006523641 A | 19 October 2006 |
| | | US 2004209943 A1 | 21 October 2004 |
| | | DE 602004018630 D1 | 05 February 2009 |
| | | IN 4711DELNP2005 A | 05 October 2007 |
| | | KR 20060011959 A | 06 February 2006 |
| | | ES 2316978 T3 | 16 April 2009 |
| | | AT 418548 T | 15 January 2009 |
| CN 101146763 A | 19 March 2008 | AU 2006229904 A1 | 05 October 2006 |
| | | US 2009054506 A1 | 26 February 2009 |
| | | WO 2006104826 A2 | 05 October 2006 |
| | | CA 2600833 A1 | 05 October 2006 |
| | | EP 1868985 A2 | 26 December 2007 |
| | | JP 2008534593 A | 28 August 2008 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110194113X


### Non-patent literature cited in the description


- **JAZAYERI A ; ANDREW S. DORÉ ; LAMB D et al.** Extra-helical binding site of a glucagon receptor antagonist [J. *Nature,* 12 May 2016, vol. 533 (7602), 274-277
- **A.GENNARO.** Remington: The Science and Practice of Pharmacy. Lippincott Williams & Wilkins, 2000
- **STRICKLEY.** *Pharmaceutical Research,* 2004, vol. 21 (2), 201-230